# EUROPEAN PATENT SPECIFICATION

(11) **EP 3 889 132 B1**
(45) Date of publication and mention of the grant of the patent: **14.12.2022**
(21) Application number: 21166820.7
(22) Date of filing: 02.04.2021
(51) Int. Cl.: C07C 233/25, C07C 233/29, A61P 25/00, A61P 35/00, A61K 31/16

(54) **MONOACYLGLYCEROL LIPASE (MAGL) INHIBITORS**
INHIBITOREN DER MONOACYLGLYCEROL LIPASE(MAGL)
INHIBITEURS DE LA LIPASE DE MONOACYLGLYCEROL (MAGL)

(30) Priority: 03.04.2020 IT 202000007150
(43) Date of publication of application: 06.10.2021
(73) Proprietor: Università di Pisa, 56126 Pisa (IT); Universita' Ca' Foscari Venezia, 30123 Venezia (VE) (IT)
(72) Inventor: GRANCHI, Carlotta, 56126 Università di Pisa, Lungarno Pacinotti 43/44 (IT); BONONI, Giulia, 56126 Università di Pisa, Lungarno Pacinotti 43/44 (IT); MACCHIA, Marco, 56126 Università di Pisa, Lungarno Pacinotti 43/44 (IT); MINUTOLO, Filippo, 56126 Università di Pisa, Lungarno Pacinotti 43/44 (IT); POLI, Giulio, 56126 Università di Pisa, Lungarno Pacinotti 43/44 (IT); SCALABRINI, Diana, 56126 Università di Pisa, Lungarno Pacinotti 43/44 (IT); TUCCINARDI, Tiziano, 56126 Università di Pisa, Lungarno Pacinotti 43/44 (IT); RIZZOLIO, Flavio, 33081 Via Montello 26, Aviano (PN) (IT); GIORDANO, Antonio, 56126 Università di Pisa, Lungarno Pacinotti 43/44 (IT)
(74) Representative: Pace Napoleone, Maria

(56) References cited:
- US-A1- 2018 134 674
- CARLOTTA GRANCHI ET AL: "Optimization of a Benzoylpiperidine Class Identifies a Highly Potent and Selective Reversible Monoacylglycerol Lipase (MAGL) Inhibitor", JOURNAL OF MEDICINAL CHEMISTRY, vol. 62, no. 4, 28 February 2019 (2019-02-28), pages 1932-1958, XP055756032, ISSN: 0022-2623, DOI: 10.1021/acs.jmedchem.8b01483

## Description

### FIELD OF THE INVENTION

The present invention relates to compounds of formula (I) useful as inhibitors, in particular as reversible non-covalent inhibitors, of the enzyme monoacylglycerol lipase (MAGL). The present invention also relates to the therapeutic use of such compounds, pharmaceutical compositions that include them and processes for obtaining them.

### BACKGROUND OF THE INVENTION

The enzyme monoacylglycerol lipase (MAGL) is a serine hydrolase expressed in most types of brain cells, including astrocytes, oligodendrocytes and microglia cells, which is part of the endocannabinoid system (ECS). The ECS is an endogenous system involved in numerous physiological and pathological processes, including neuroprotection, pain perception, anxiety, immune modulation, motor coordination, inflammation and the regulation of neoplastic cells. The ECS consists of two G protein-coupled receptors called cannabinoid receptor type 1 and 2 (CB1 and CB2), endogenous lipid molecules called endocannabinoids (eCBs) and numerous enzymes responsible for the synthesis and degradation of such molecules. CB1 receptors are found mainly in the central nervous system (CNS), while CB2 are predominantly expressed on T cells of the immune system, but their presence has been recently demonstrated also in the CNS. The eCBs act as signal molecules that exert their biological actions in the human body by interacting with the CB1 and CB2 receptors. The main eCB present in the brain is 2-arachidonoylglycerol (2-AG), which after activating cannabinoid receptors is transported into the cell cytoplasm to be degraded. MAGL is the enzyme responsible for the hydrolysis of 85% of 2-AG, while the remaining 15% is hydrolysed by two other enzymes, ABHD6 and ABHD12. Numerous studies have demonstrated the therapeutic potential of MAGL for the treatment of various types of pathologies. In fact, the reduced degradation of 2-AG obtained with the inhibition of the catalytic activity of MAGL represents a promising pharmacological strategy to activate the ECS without incurring the adverse effects typically associated with the direct activation of cannabinoid receptors, such as hypomotility, hypothermia and catalepsy. It has been demonstrated that the genetic or pharmacological depletion of MAGL activity has a neuroprotective effect in various animal models of demyelinating diseases, such as multiple sclerosis and other neurodegenerative diseases. Furthermore, the hydrolysis of 2-AG carried out by MAGL leads to the formation of arachidonic acid (AA), which is the precursor of pro-inflammatory eicosanoids (prostaglandins, prostacyclines, thromboxanes and leukotrienes). For this reason, in vivo studies of inhibition of the MAGL enzyme have shown that a drastic reduction of hydrolytic activity against 2-AG leads to a decreased formation of prostaglandins and thromboxanes deriving from AA, thus highlighting the key role of MAGL in the regulation of inflammatory processes. Finally, various studies have demonstrated that MAGL has an important role in the development of invasive tumours, being involved in lipid metabolism, which has been considered a fundamental target in the treatment of tumours. In fact, tumour cells need a large pool of fatty acids, both for the synthesis of new cell membranes of rapidly growing cells and for the formation of tumorigenic cell mediators. In this context, MAGL results to have a key role because, in addition to metabolize 2-AG at the CNS level, it also hydrolyzes monoacylglycerols to fatty acids and glycerol at the peripheral level, thus providing cancer cells with a significant amount of lipid substrates.

Despite the development of potent MAGL inhibitors with different chemical structures in recent years, their number is still quite limited. Furthermore, most of these compounds are characterized by irreversible inhibition of the enzyme. Compounds with irreversible behaviour have generated important side effects in animal models and have therefore represented an obstacle for in vivo studies and also for any clinical trials. The permanent inactivation of MAGL can in fact lead to an excess of 2-AG, which is not metabolized, determining a continuous activation of the brain CB1 receptors. This, in turn, causes a desensitization of the CB1 receptors, a functional antagonism of the ECS that leads to a progressive loss of the therapeutic effect, accompanied by addiction phenomena. The reference MAGL inhibitor that has been used in the literature for most cell and animal experiments is the carbamate molecule JZL184, reported by Cravatt in 2009. This compound shows nanomolar inhibitory activity on the isolated enzyme (IC₅₀ = 4 nM reported in literature, 48.6 nM under the conditions of Example 2). Another carbamate nanomolar inhibitor frequently used as a reference for MAGL inhibition studies is the compound CAY10499 reported by Muccioli and Lambert in 2008.

A solution to the onset of these side effects consists in the use of MAGL inhibitors characterized by a reversible behaviour, which is a still partially unexplored area of research. Contrary to irreversible inhibition, reversible inhibitors would only produce a temporary inhibition of MAGL, thus leaving the basal functioning of the ECS intact. This approach has not yet been used for in vivo studies, mainly due to the unavailability of effective reversible MAGL inhibitors.

However, in 2014 Hernandez-Torres and co-workers identified the first compound characterized by a reversible non-covalent interaction with MAGL. This inhibitor was tested in vivo using the experimental allergic encephalomyelitis (EAE) murine model, which is extensively studied as an animal model of CNS demyelinating diseases such as multiple sclerosis and acute disseminated encephalomyelitis. This compound showed a MAGL inhibition activity in the micromolar range (IC₅₀ = 9.0 µM under the conditions of Example 2). Although this molecule is not a very powerful MAGL inhibitor, it has been shown that this compound is capable of improving the clinical progression of the murine model of multiple sclerosis without causing catalepsy or other side effects that were instead observed after the administration of irreversible MAGL inhibitors. Furthermore, Granchi et al (Journal of Medicinal Chemistry, vol 62, no.4, 2019, pages 1932-1958) describes benzoylpiperidine derivatives as reversible MAGL inhibitors. These results therefore support the hypothesis that reversible MAGL inhibitors can be used profitably for the treatment of various diseases. The development of effective reversible MAGL inhibitors therefore represents a research area of wide interest. Therefore, there is a need to develop further MAGL inhibitors, which are powerful and at the same time ideally endowed with a reversible mechanism of action.

### SUMMARY OF THE INVENTION

The present invention relates to compounds of formula (I), as defined below. In the present invention, it was surprisingly found that such compounds act as powerful inhibitors of the enzyme monoacylglycerol lipase (MAGL) with a reversible mechanism of action, mediated in particular by non-covalent bonds.

In vitro screening for the evaluation of the enzymatic inhibition of MAGL demonstrated activity in the nanomolar range for the compounds of the invention, comparable to those of the most active MAGL inhibitors currently present in the literature.

Reversibility tests and kinetic evaluations confirmed the reversible inhibition mechanism of these molecules. This mechanism is advantageous as it could avoid the onset of the known side effects generated by irreversible MAGL inhibitors.

The compounds of the invention can be advantageously used to prevent and/or treat any condition that could be improved by the inhibition of MAGL, such as serious pathological conditions of neurodegenerative inflammation and tumours. In fact, the compounds of formula (I) resulted to be effective in inhibiting the cell viability of various tumour cell lines, such as colorectal cancer, breast cancer and ovarian cancer.

### DETAILED DESCRIPTION OF THE INVENTION

Therefore, a compound of formula (I) constitutes an object of the present invention: 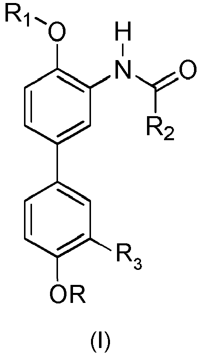 in which:
R and R₁ are independently selected from the group consisting of: H, linear C1-C6 alkyl chain, benzyl and acetyl;
R₂ is selected from the group consisting of: linear C1-C20 alkyl chain, branched C3-C10 alkyl chain, aryl, heteroaryl and linear C1-C10 alkyl chain substituted by aryl or heteroaryl;
R₃ is selected from the group consisting of: halogen, haloalkyl and CN;
the two rings shown are optionally and independently of each other substituted with one or more substituents independently selected from the group consisting of: halogen, linear or branched C1-C6 alkyl chain, haloalkyl, haloalkoxyl, CN, NO₂, NH₂, carboxylic ester, COOH and SH;
or a salt, stereoisomer, solvate or tautomer thereof.

The preferred realization forms reported below can be combined with each other in any way that gives rise to a chemically stable structure.

Preferably, this salt, stereoisomer, solvate or tautomer is pharmaceutically acceptable. Preferably, at least one linear C1-C6 alkyl chain independently of the others is a linear C1-C4 alkyl chain, preferably it is methyl. Preferably this linear C1-C6 alkyl chain in position R and/or R₁ is methyl.

Preferably, this linear C1-C20 alkyl chain is a linear C4-C15 alkyl chain.

Preferably, this C3-C6 branched alkyl chain is isopropyl or tert-butyl.

Preferably, at least one aryl independently of the others is phenyl or naphthyl.

Preferably, at least one halogen independently of the others is F, Cl or Br, preferably it is F. Preferably, at least one haloalkyl independent of the others is CF₃.

Preferably, this haloalkoxyl is OCF₃.

Preferably, this carboxylic ester is COOMe.

Preferably, R is H or methyl. Preferably, R is H.

Preferably, R₃ is selected from the group consisting of: F, Cl, Br, CF₃ and CN. Preferably, R₃ is F.

Preferably, R is H and R₃ is F.

Preferably, R₁ is H or methyl. Preferably, R₁ is H.

Preferably, R₂ is selected from the group consisting of: (CH₂)ₚCH₃, CMeR₃R₄ and (CH₂)_{q}Ph, wherein: p is an integer comprised between 0 and 19, R₃ and R₄ are independently of each other H or Me, q is an integer comprised between 1 and 10. Preferably, p is an integer comprised between 3 and 14. Preferably, R₃ is hydrogen and R₄ is methyl or R₃ and R₄ are both methyl. Preferably, q is an integer comprised between 1 and 4.

Preferably, R₂ is selected from the group consisting of: n-butyl, n-pentyl, n-hexyl, n-heptyl, n-octyl, n-nonyl, n-undecanyl, n-tridecanyl, n-pentadecanyl, isopropyl, tert-butyl, phenyl, benzyl, (CH₂)₂phenyl, (CH₂)₃phenyl, (CH₂)₄phenyl and (CH₂)naphthyl.

In a preferred embodiment, the compound, salt, stereoisomer, solvate or tautomer of the present invention is of formula (II): 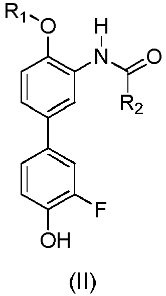 wherein R₁ and R₂ are as defined above.

In a further preferred embodiment, the compound, salt, stereoisomer, solvate or tautomer of the present invention is of formula (III): 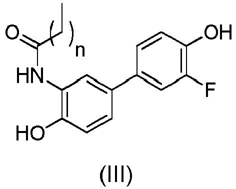 where n is an integer comprised between 0 and 19. Preferably, n is an integer comprised between 3 and 14.

In a further preferred embodiment, the compound, salt, stereoisomer, solvate or tautomer of the present invention is of formula (IV): 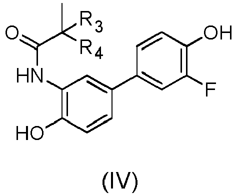 in which R₃ and R₄ are independently of each other hydrogen or linear or branched C1-C8 alkyl chain (for example methyl). Preferably, R₃ is hydrogen and R₄ is methyl or R₃ and R₄ are both methyl.

In yet another preferred embodiment, the compound, salt, stereoisomer, solvate or tautomer of the present invention is of formula (V): 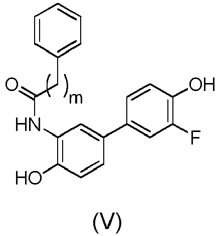 wherein m is an integer comprised between 1 and 10. Preferably, m is an integer comprised between 1 and 4.

Preferably, the compound of the present invention is selected from the group consisting of:

| | | | | | |
|---|---|---|---|---|---|
| 1 | 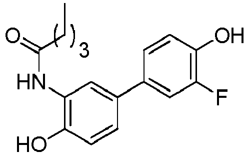 | | | 9 | 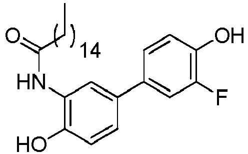 |
| 2 | 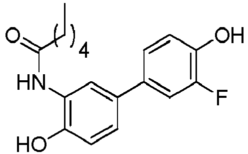 | | | 10 | 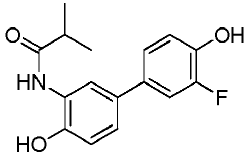 |
| 3 | 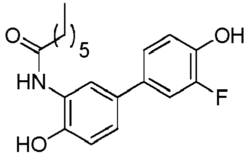 | | | 11 | 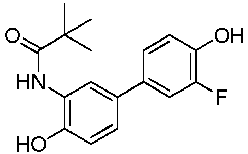 |
| 4 | 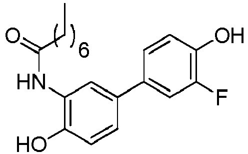 | | | 12 | 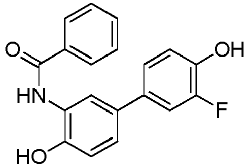 |
| 5 | 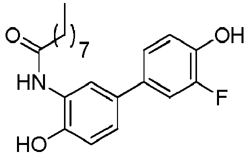 | | | 13 | 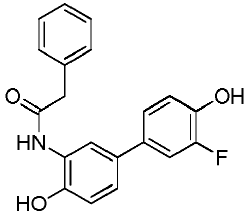 |
| 6 | 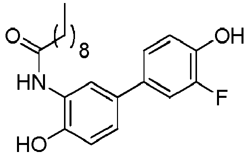 | | | 14 | 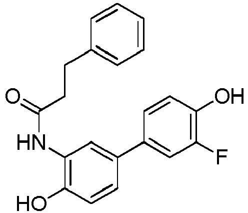 |
| 7 | 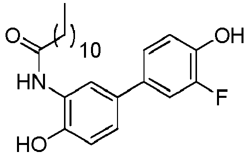 | | | 15 | 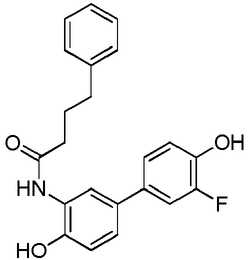 |
| 8 | 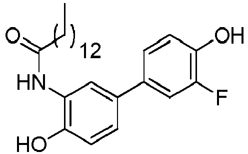 | | | 16 | 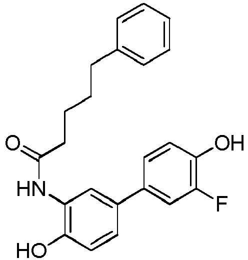 |
| | | 17 | 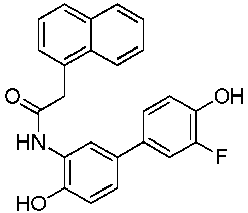 | | |

or it is a salt, stereoisomer, solvate or tautomer of them.

In the following aspects of the invention, the term "compound" also includes any salt, stereoisomer, solvate or tautomer thereof.

In one aspect, the compound as defined above is an inhibitor of the enzyme monoacylglycerol lipase (MAGL). Preferably, this compound is a reversible inhibitor of the MAGL enzyme. Preferably, this compound is a reversible and/or non-covalent and/or competitive inhibitor of the MAGL enzyme. Therefore, the compounds of the present invention can be used in therapy, but also in biological, cellular, biochemical assays, etc.

In a preferred aspect, the present invention provides an inhibitor of the MAGL enzyme, in particular a reversible and/or non-covalent and/or competitive inhibitor, having the structure defined above.

In a further aspect, the present invention provides a method, in particular an in vitro method, for inhibiting the MAGL enzyme comprising the step of binding this enzyme with a compound as defined above. This method may include the further step of comparing the results to an appropriate control and can therefore be used to assess whether a given compound acts as an inhibitor/activator of the MAGL enzyme.

In one aspect, the compound as defined above is for use as a medicament. Preferably, this medicament is neuroprotective, analgesic, anti-inflammatory, antitumour and/or anxiolytic. Preferably, such medicament is an inhibitor of the MAGL enzyme.

In a further aspect, the compound as defined above is for use as an inhibitor of the enzyme monoacylglycerol lipase (MAGL).

Preferably, this inhibitor of the enzyme monoacylglycerol lipase (MAGL) is a reversible inhibitor. Preferably, this inhibitor of the enzyme monoacylglycerol lipase (MAGL) is a reversible and/or non-covalent and/or competitive inhibitor.

In yet a further aspect, the compound as defined above is for use in the prevention and/or prophylaxis and/or treatment of any condition that could be prevented, cured, improved and/or eradicated by inhibition of the MAGL enzyme, such as for example a MAGL-mediated condition, a disease in which the MAGL enzyme is overexpressed/upregulated or a disease characterized by an activity of the MAGL enzyme higher than in healthy subjects.

Preferably, this condition is selected from the group consisting of: tumour, neuroinflammation, a neurodegenerative disease, pain, a neurological disorder, a psychiatric disorder, a head injury, an autoimmune disease, inflammation, abstinence, a metabolic disorder, steatohepatitis, and any combination of them.

Preferably, this condition is a pathological condition of neurodegenerative inflammation or a tumour.

Preferably, this tumour is selected from the group consisting of: colorectal cancer, breast cancer, ovarian cancer, pancreatic cancer, liver cancer and any combination thereof. Preferably, this tumour is a carcinoma or an adenocarcinoma. Preferably, this tumour is primary or metastatic. Preferably, this tumour prevention and/or treatment includes the prevention and/or treatment of at least one tumour symptom, such as nausea and pain. Preferably, this neurodegenerative disease is selected from the group consisting of: Alzheimer's disease, Parkinson's disease, Huntington's disease, a demyelinating disease, such as multiple sclerosis, amyotrophic lateral sclerosis and acute disseminated encephalomyelitis, and any combination thereof.

Preferably, this pain is acute or chronic.

Preferably, this neurological disorder is selected from the group consisting of: epilepsy, migraine, neurotoxicity and stroke (including cerebral haemorrhage and ischemia). Preferably, this psychiatric disorder is anxiety or depression.

Preferably, this metabolic disorder is the metabolic syndrome.

Preferably, this steatohepatitis is non-alcoholic steatohepatitis.

The present invention also provides the combination of the compound of the invention with a further therapeutic intervention, such as for example radiotherapy or chemotherapy. Therefore, the use as defined above is preferably in combination with a further therapeutic intervention, such as radiotherapy or chemotherapy. Preferably, this further therapeutic intervention is a further therapeutic agent. When this further therapeutic intervention is a further therapeutic agent, the combination can be formulated in a single dosage form or the compound of the invention and the further therapeutic agent can be administered separately, both simultaneously and sequentially.

Therefore, a further object of the present invention is constituted by a pharmaceutical composition comprising:
- the compound, salt, stereoisomer, solvate or tautomer as defined above,
- a pharmaceutically acceptable carrier, and
- optionally a further therapeutic agent.

Preferably, this further therapeutic agent is one or more of: a modulator of the cannabinoid system, a neuromodulator, an anti-inflammatory.

In one aspect, this composition is for use in the prevention and/or treatment of any condition as defined above.

A further object of the present invention is a process for the preparation of a compound, salt, stereoisomer, solvate or tautomer as defined above in which R and/or R₁ are H. Preferably, this process comprises the deprotection step of a compound of formula (VI): 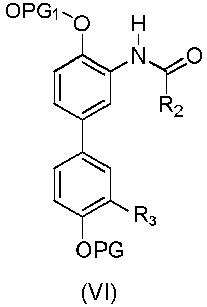 wherein PG and PG₁ are the same or different protecting groups to obtain a compound of formula (I), salt, stereoisomer, solvate or tautomer as defined above wherein R and/or R₁ are H. Suitable deprotection conditions can be selected from technician of the sector based on the protecting groups used. Preferably, this protecting groups are independently selected from each other from the group consisting of: benzyl, acetyl and linear C1-C6 alkyl chain, for example methyl. When this protecting group is a C1-C6 linear alkyl chain, the deprotection can be carried out according to the standard methods used in the sector, for example in the presence of BBr₃ and/or in DCM and/or from a temperature below 0 °C up to room temperature. In one aspect, the compound of formula (VI) can be prepared by a Suzuki reaction between an aryl-boronic acid of formula (VII) and an aryl halide of formula (VIII) under the standard conditions used in the field: 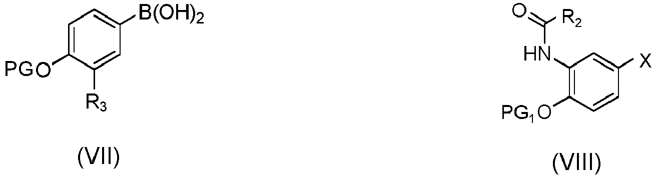 where X is a halogen.

The compounds of formula (VII) and (VIII) are commercial or can be prepared by standard methods well known to technician of the sector.

As used herein, "alkyl chain" or "alkyl" refers to a straight or branched hydrocarbon chain consisting exclusively of carbon and hydrogen atoms linked by single bonds, containing the specified number of carbon atoms. Preferably, this alkyl chain contains from 1 to 20 carbon atoms (C1-C20) or from 1 to 15 carbon atoms (C1-C15) or from 1 to 6 carbon atoms (C1-C6) or from 1 to 4 atoms carbon (C1-C4). It should be understood that when this alkyl chain is branched, the minimum number of carbon atoms is 3. Examples of alkyl chain include but are not limited to methyl, ethyl, n-propyl, isopropyl, butyl, sec-butyl, tert-butyl, pentyl, isopentyl, tert-pentyl, hexyl, heptyl, octyl, nonyl, decanyl, dodecanyl, tridecanyl, tetradecanyl, pentadecanyl, etc.

As used herein, the term "aryl" refers to a monocyclic, bicyclic or polycyclic aromatic system, consisting exclusively of carbon and hydrogen atoms. Suitable examples of aryl are phenyl and naphthyl. As used herein, the term "heteroaryl" refers to an aryl in which at least one carbon atom is replaced with a heteroatom.

As used herein, the term "haloalkyl" refers to a linear or branched alkyl chain, as defined above, in which at least one hydrogen atom is replaced by a halogen. Preferably, the length of the alkyl chain in this haloalkyl is: from 1 to 20 carbon atoms (C1-C20 haloalkyl) or from 1 to 15 carbon atoms (C1-C15 haloalkyl) or from 1 to 6 carbon atoms (C1- C6 haloalkyl) or from 1 to 4 carbon atoms (C1-C4 haloalkyl). Suitable examples of haloalkyl include CF₃, CH₂CH₂CH₂F, C₄F₉, CHF₂, CH₂F.

As used herein, the term "haloalkoxyl" refers to a linear or branched alkyl chain, as defined above, linked via an oxygen atom in which at least one hydrogen atom is replaced by a halogen. Preferably, the length of the alkyl chain in this haloalkoxy is: from 1 to 20 carbon atoms (C1-C20 haloalkoxyl) or from 1 to 15 carbon atoms (C1-C15 haloalkoxyl) or from 1 to 6 carbon atoms (C1- C6 haloalkoxy) or from 1 to 4 carbon atoms (C1-C4 haloalkoxyl).

As used herein, the term "carboxylic ester" refers to the -COOR' functionality, in which R' is preferably an alkyl chain as defined above.

Within the scope of the present invention, the salts of the compounds defined above are also included in all possible stoichiometric and non-stoichiometric forms. As used herein, the term "salt" refers to derivatives of the compounds of the invention in which the compound of the invention is modified by converting an acid or basic group into the corresponding addition salt with a base or a conventional acid, respectively. Due to their potential use in medicine, such salts are preferably pharmaceutically acceptable. Pharmaceutically acceptable salts include conventional non-toxic salts obtained by salification of a compound of formula (I) with inorganic acids (e.g., hydrochloric, hydrobromic, sulfuric or phosphoric acid), or with organic acids (e.g., acetic, propionic, succinic, benzoic, sulphanilic, 2-acetoxy-benzoic, cinnamic, mandelic, salicylic, glycolic, lactic, oxalic, malic, maleic, malonic, fumaric, tartaric, citric, p-toluenesulfonic, methanesulfonic, ethanesulfonic, or naphthalenesulfonic). Other suitable pharmaceutically acceptable salts include pharmaceutically acceptable alkali or alkaline earth metal salts such as sodium, potassium, calcium or magnesium salts. For a review of suitable pharmaceutical salts see: Berge S. M. et al., J. Pharm. Sci. 1977, 66, 1-19; Gould P. L. Int. J. Pharm 1986, 33, 201-217 and Bighley et al. Encyclopedia of Pharmaceutical Technology, Marcel Dekker Inc, New York 1996, Volume 13, page 453-497. Other salts, which are not pharmaceutically acceptable, for example the trifluoroacetate salt, can be useful in the preparation of compounds of the present invention and these salts constitute a further aspect of the invention. Advantageously, the salts of the invention can be more stable than the corresponding compounds in free form.

Furthermore, the compounds of the invention can exist in non-solvated forms as well as in solvated forms, in particular with pharmaceutically acceptable solvents such as water, EtOH and analogues. Such solvated forms constitute a further object of the present invention. Some compounds of the invention may exist in stereoisomeric forms, i.e., they may contain one or more asymmetric centers, in particular asymmetric carbon atoms. The optically pure single stereoisomers (enantiomers and diastereomers) as well as the mixtures (racemic and not-racemic) of these are included in the scope of the present invention. The mixtures of stereoisomers can be separated to give a single enantiomer e.g., using preparative HPLC with a chiral stationary phase column or resolved to produce single enantiomers using methods known to technicians of the sector. Furthermore, chiral intermediates can be resolved and used to prepare single enantiomers.

Similarly, it should be understood that the compounds of the invention may exist in tautomeric forms other than those shown in the formulas represented here. Such tautomeric forms are also included within the scope of the present invention.

The invention also includes all isotopic variations of the compounds of the invention. An isotopic variation is defined as the one in which at least one atom is replaced by an atom having the same atomic number but an atomic mass different from the atomic mass usually found in nature.

Examples of isotopes that can be incorporated into the compounds of the invention include isotopes such as ²H, ³H, ¹³C, ¹⁴C, ¹⁵N, ¹⁷O, ¹⁸O, ³¹P, ³²P, ³⁵S, ¹⁸F e ³⁶C, respectively. Substitution with isotopes such as deuterium ²H may provide certain therapeutic benefits resulting from greater metabolic stability. The isotopic variations of the compounds of the invention can generally be prepared by conventional procedures as well as by the illustrative methods or with the preparations described in the examples below using suitable isotopic variations of the suitable reagents.

It will be appreciated by the technicians of the sector that certain protected derivatives of the compounds of the invention can act as prodrugs, i.e., they do not possess pharmacological activity as such, but can be administered orally or parenterally and subsequently metabolized in the organism to form compounds of the invention that are pharmacologically active. All the prodrugs of the compounds defined above are equivalent to the compounds of formula (I) and are therefore included in the scope of the invention. Examples of prodrugs suitable for the compounds of the present invention are described in Drug of Today, Volume 19, Number 9, 1983, pp 499-538, in Topics in Chemistry, Chapter 31, pp 306-316, in "Design of Prodrugs" by H. Bundgaard, Elsevier, 1985, Chapter 1 and also include compounds of formula (VI), in particular compounds of formula (VI) in which PG and PG₁ are independently of each other benzyl, acetyl or linear C1-C6 alkyl chain such as methyl. It will be further appreciated by technicians of the sector that certain portions, known to the experts as "pro" portions, described by H. Bundgaard, in "Design of Prodrugs", can be positioned on appropriate features when such features are present within the compounds of the invention.

The compounds described so far can be in crystalline, co-crystalline, amorphous or polymorphic form and any of them are part of the invention.

The monoacylglycerol lipase (MAGL) enzyme, also known as MGL or MGLL, is well known in the sector. The terms "monoacylglycerol lipase", MAGL, MGL and MGLL are used interchangeably herein. As used herein, "MAGL" includes the protein (enzyme) as well as any fragment, derivative, variant, isoform, polymorph, etc. of it, especially the functional ones. In the present invention, MAGL is preferably human. Preferably, the amino acid sequence of MAGL is one of those defined in the Universal Protein Resource database (UniProtKB) at number Q99685 in the version of 15 October 2019 (https://www.uniprot.org/uniprot/Q99685). By "inhibition of MAGL" or grammatical variants of this expression, is to be meant in particular the inhibition of at least one function of MAGL, for example: the catalytic activity of MAGL in the hydrolysis of 2-arachidonoylglycerol (2-AG) and of other lipid molecules and the ability of MAGL to bind 4-nitrophenylacetate (4-NPA).

The inhibition of MAGL can be measured according to any technique known in the sector, for example by calculating the IC₅₀ (concentration of inhibitor which reduces the activity of MAGL by 50% compared to its activity in the absence of inhibitor) or of *K*ᵢ (inhibition constant) as described in the Examples below. Preferably, the compounds of the invention show an IC₅₀ for MAGL measured as described in the assays below equal to or less than 25.0 µM, more preferably, equal to or less than 20 µM, even more preferably, less than 10 µM, in particular less than 8 µM.

MAGL inhibition can also be measured by the assays described in Deng Hui, Li Weimin, Monoacylglycerol lipase inhibitors: cancer malignancy, neurological and metabolic disorders, Acta Pharmaceutica Sinica B, https://doi.org/10.1016/j.apsb.2019.10.006, which is incorporated herein as a reference.

The MAGL inhibition can be measured with respect to an appropriate control, for example the isolated enzyme not treated in any way, a healthy subject, a subject affected by a pathology mediated by the MAGL activity, the isolated enzyme treated with a MAGL inhibitor, a subject during the course of treatment with a MAGL inhibitor.

Preferably, the compounds of the present invention inhibit the activity of MAGL by about 10%, 20%, 30%, 40%, 50%, 60%, 70%, 80%, 90% with respect to an appropriate control.

The term "inhibition" or grammatical variants of this expression includes any degree of decrease of this function with respect to an appropriate control, including eradication.

By "inhibition" or grammatical variants of this expression, is to be meant in particular the establishment of a link between MAGL and an inhibitor, which slows down or inhibits the speed of an enzymatic reaction compared to the speed of the same reaction in the absence of the inhibitor.

Reversible inhibition is characterized by the fact that the link between MAGL and the inhibitor can break and the enzymatic reaction can proceed. In the case of a reversible type of inhibition, the inhibitory activity of the molecule remains almost unchanged by applying different ligand-enzyme co-incubation times. (Granchi C, et al., Optimization of a Benzoylpiperidine Class Identifies a Highly Potent and Selective Reversible Monoacylglycerol Lipase (MAGL) Inhibitor. J Med Chem. 2019 Feb 28; 62 (4): 1932-1958. Doi: 10.1021 / acs. jmedchem.8b01483. Epub 2019 Feb 11. PMID: 30715876.)

By "non-covalent inhibition" or grammatical variants of this expression, is to be meant in particular the establishment of a link between MAGL and an inhibitor mediated by non-covalent interactions, such as hydrogen bond, hydrophobic interaction and ionic bond. In the case of covalent inhibition, the compound is usually able to covalently bind the cysteine residues. To evaluate this possibility, it is possible to measure the inhibitory activity in the presence of 1,4-Dithio-DL-threitol (DTT). A decrease in the inhibitory activity of the molecule in the presence of DTT leads to a covalent inhibition. (Granchi C, et al., Tuccinardi T. Structural Optimization of 4-Chlorobenzoylpiperidine Derivatives for the Development of Potent, Reversible, and Selective Monoacylglycerol Lipase (MAGL) Inhibitors. J Med Chem. 2016 Nov 23; 59 (22): 10299- 10314. doi: 10.1021/acs.jmedchem.6b01459. Epub 2016 Nov 14. PMID: 27809504.) A competitive inhibitor is an enzyme inhibitor that follows a competitive inhibition mechanism, i.e., competes with the substrate for binding to the active site of the enzyme. Once the Michaelis-Menten inhibition curve has been obtained and the values of Kₘ and Vₘₐₓ have been calculated, it is possible to calculate the value of α. Its value is greater than zero and determines to what extent the binding process of the inhibitor changes the affinity of the enzyme for the substrate. When α is very high, inhibitor binding prevents substrate binding and competitive inhibition occurs. (Granchi C, et al., Structural Optimization of 4-Chlorobenzoylpiperidine Derivatives for the Development of Potent, Reversible, and Selective Monoacylglycerol Lipase (MAGL) Inhibitors. J Med Chem. 2016 Nov 23; 59 (22): 10299-10314. Doi : 10.1021 / acs.jmedchem.6b01459. Epub 2016 Nov 14. PMID: 27809504.)

As used herein, the term "treatment" or grammatical variants thereof includes: (1) inhibition of the condition (for example, stopping, reducing or delaying the development of the condition or a relapse or at least one symptom of the condition); and/or (2) relieve the condition (including regression of the condition or at least one of its symptoms); and/or (3) keep the condition stable to avoid worsening. The benefit for a patient to be treated is statistically significant or at least perceptible to the patient or physician. However, it will be appreciated that when a compound of the invention is administered to a patient to treat a condition, the result may not always be a complete treatment.

As used herein, the terms "prevention" and "prophylaxis" or grammatical variants of them include: the prevention or delay of the onset of at least one symptom of the condition in a subject at risk of being affected or predisposed to the condition but not yet showing any symptoms of the condition.

"Neuroinflammation", as used herein, refers for example to acute or chronic inflammation of the nervous tissue, which is the major component of the nervous system tissue, i.e., the brain, spinal cord and branched peripheral nerves of the peripheral nervous system (PNS). Chronic neuroinflammation is associated with neurodegenerative diseases such as Alzheimer's disease, Parkinson's disease and multiple sclerosis. Acute neuroinflammation usually immediately follows an injury to the central nervous system, such as following a head injury.

The term "head injury" as used herein refers for example to damage to the brain resulting from an external mechanical force, such as rapid acceleration, deceleration, impact, explosive waves or penetration.

The term "neurodegenerative disease", as used herein, refers for example to diseases related to the progressive loss of structure or function of neurons, including the death of neurons, or of a part of them, such as the myelin sheath. Examples of neurodegenerative diseases include, by way of example, Alzheimer's disease, Parkinson's disease and demyelinating diseases, such as amyotrophic lateral sclerosis, multiple sclerosis and acute disseminated encephalomyelitis.

The term "psychiatric disorder", otherwise known as "mental illness", refers for example to behavioural or mental patterns that can cause the individual to suffer or to function poorly in life. These features can be persistent, relapsing and remitting, or occur as a single episode. Examples of mental disorders include, by way of example, anxiety and depression.

The term "neurotoxicity", as used herein, specifically refers to toxicity in the nervous system. It occurs when exposure to natural or man-made toxic substances (neurotoxins) alters the normal activity of the nervous system in a way that causes damage to the nerve tissue. Examples of neurotoxicity include, by way of example, neurotoxicity resulting from exposure to substances used in chemotherapy, radiation therapy, drug abuse and organ transplants, as well as exposure to heavy metals, certain foods and food additives, pesticides, solvents and/or industrial detergents, cosmetics and some naturally occurring substances.

The term "tumour", as used herein, is interchangeable with the term "cancer" and refers in particular to a disease characterized by the presence of a neoplasm resulting from an uncontrolled abnormal growth of cells (such cells are "tumour cells").

"Pain", as used herein, includes chronic (including idiopathic pain) or acute pain and also pain-related conditions, such as neuropathic pain (including diabetic polyneuropathy), nociceptive pain, persistent pain, osteoarthritic pain, pain due to cancer and/or chemotherapy (e.g., neuropathy due to chemotherapy), inflammatory pain, postoperative pain, fibromyalgia, chronic widespread pain, musculoskeletal pain, myofascial pain, temporomandibular joint pain (TMJ pain), pain associated spasticity.

The pharmaceutical compositions of the present invention can be selected according to the treatment needs. These compositions are prepared by mixing and are suitably adapted to the chosen route of administration. Any pharmaceutical composition or formulation of the compounds described so far is included within the scope of the present invention.

The routes of administration of the pharmaceutical compositions of the present invention include parenteral, oral, pulmonary, ophthalmic, nasal (inhalation), rectal, vaginal, aural, topical, buccal, transdermal, intravenous, intramuscular, subcutaneous, intradermal, intraocular, intracerebral, intralymphatic, intraarticular, intrathecal and intraperitoneal.

For oral administration, the pharmaceutical compositions of the present invention can be for example in the form of tablets, capsules, oral preparations (including liquid), powders, granules, pills. The solid oral compositions can be prepared by conventional mixing, filling or tableting methods. The mixing operation can be repeated to distribute the active ingredient in all compositions containing large quantities of fillers. Such operations are conventional.

Tablets and capsules for oral administration are generally presented in unit dosage forms and contain conventional excipients such as binders, fillers (including cellulose, mannitol, lactose), diluents, tablet agents, lubricants (including magnesium stearate), detergents, disintegrants (for example polyvinylpyrrolidone and starch derivatives such as sodium starch glycolate), dyes, flavouring and wetting agents (for example sodium lauryl sulfate).

The oral liquid preparations can be in the form of, for example, aqueous or oily suspensions, solutions, emulsions, syrups or elixirs, or they can be presented as a dry product to be reconstituted with water or a suitable vehicle before use. Such liquid preparations can contain conventional additives such as suspending agents, for example sorbitol, syrup, methylcellulose, gelatin, hydroxyethylcellulose, carboxymethylcellulose, aluminum stearate gel or hydrogenated edible fats; emulsifying agents, e.g., lecithin, sorbitan monooleate, or acacia; non-aqueous vehicles (which may include edible oils) such as almond oil, fractionated coconut oil, oil esters such as glycerin esters, propylene glycol, or ethyl alcohol; preservatives, such as methyl or propyl p-hydroxybenzoate or sorbic acid, and if desired, conventional flavourings or dyes. The oral formulations also include conventional slow-release formulations such as coated gastro-resistant tablets or granules.

The pharmaceutical preparation for administration by inhalation can be provided by an insufflator or a pressure nebulizer.

For parenteral administration the compound can be suspended or dissolved, depending on the vehicle and concentration. Parenteral solutions are normally prepared by dissolving the compound in a vehicle, sterilizing the latter by filtration, filling suitable bottles and sealing. Advantageously, adjuvants such as local anaesthetics, preservatives and buffering agents can be dissolved in the vehicle. To increase stability, the composition can be frozen after filling the vials and removing the water under vacuum. The parenteral suspensions are prepared in substantially the same way, except that the compound can be suspended in the vehicle instead of being dissolved, and sterilized by exposure to ethylene oxide prior to suspension in the sterile vehicle. Conveniently, a surfactant or a wetting agent can be included in the composition to facilitate uniform distribution of the compound of the invention. The compositions for parenteral administration can be infused or injected.

For rectal administration, the compositions can be for example enemas or suppositories containing conventional bases such as cocoa butter, polyethylene glycol, or other glycerides. Another route of administration of the compounds of the present invention concerns topical treatment. Topical formulations may contain for example ointments, creams, lotions, gels, solutions, pastes and/or may contain liposomes, micelles and/or microspheres. Examples of ointments include oily ointments such as vegetable oils, animal fats, semisolid hydrocarbons, emulsifiable ointments such as hydroxystearin sulfate, anhydrous lanolin, hydrophilic petrolatum, cetyl alcohol, glycerol monostearate, stearic acid, water-soluble ointments containing polyethylene glycols of various molecular weights. Creams, as known to formulation experts, are viscous liquids or semi-solid emulsions, and contain an oil phase, an emulsifier and an aqueous phase. The oily phase generally contains petrolatum and an alcohol such as cetyl or stearic alcohol. Formulations suitable for topical ocular administration also include eye drops, in which the active ingredient is dissolved or suspended in a suitable vehicle, in particular in an aqueous solvent for the active ingredient.

A further method of administration of the compounds of the invention relates to transdermal delivery. Typical transdermal formulations include conventional aqueous and non-aqueous carriers, such as creams, oils, lotions or pastes or may be in the form of medicated membranes or patches.

In order to increase bioavailability, the compounds can be pharmaceutically formulated into liposomes or nanoparticles. Acceptable liposomes can be neutral, negatively or positively charged, with the charge being a function of the charge of the liposome components and the pH of the liposomal solution. Liposomes can normally be prepared using a mixture of phospholipids and cholesterol. Suitable phospholipids include phosphatidylcholine, phosphatidylethanolamine, phosphatidic acid, phosphatidylglycerol, phosphatidylinositol. Polyethylene glycol can be added to improve the blood circulation time of liposomes.

Acceptable nanoparticles include albumin nanoparticles and gold nanoparticles.

A reference for the formulations is Remington's book (Remington "The Science and Practice of Pharmacy", Lippincott Williams & Wilkins, 2000).

As used herein, the term "vehicle", used interchangeably with "excipient", includes any pharmaceutically inactive and non-toxic organic or inorganic adjuvant, such as: diluents, absorbents, adsorbents, lubricants, binders, disintegrants, surfactants, dyes, sweeteners, antioxidants, antimicrobials, polymers, preservatives, solvents, solubilizers, isotonic agents, substances that increase viscosity, stabilizers, wetting agents, emulsifiers, sweeteners, flavourings, salts to vary the osmotic pressure, buffers, soothing or masking agents. Examples of excipient include lactose, sucrose, D-mannitol, starch, corn starch, crystalline cellulose, anhydrous silicic acid and similar substances.

Examples of lubricants include magnesium stearate, calcium stearate, talc, colloidal silica and similar substances.

Examples of binder include crystalline cellulose, white sugar, D-mannitol, dextrin, hydroxypropylcellulose, hydroxypropylmethylcellulose, polyvinylpyrrolidone, starch, sucrose, gelatin, methylcellulose, sodium carboxymethylcellulose and similar substances.

Examples of disintegrant include starch, carboxymethylcellulose, calcium carboxymethylcellulose, sodium carboxymethyl starch, L-hydroxypropylcellulose and similar substances.

Examples of solvents include water for injections, alcohol, propylene glycol, macrogol, sesame oil, corn oil, olive oil and similar substances.

Examples of solubilizer include polyethylene glycol, propylene glycol, D-mannitol, benzyl benzoate, ethanol, trisaminomethane, cholesterol, triethanolamine, sodium carbonate, sodium citrate and similar substances.

Examples of surfactants include stearyl triethanolamine, sodium lauryl sulfate, laurylaminopropionic acid, lecithin, benzalkonium chloride, benzethonium chloride, glycerin monostearate and the like, hydrophilic polymers such as polyvinyl alcohol, polyvinylpyrrolidone, sodium carboxymethylcellulose, hydroxycellulose, hydroxycellulose, hydroxycellulose, hydroxycellulose, hydroxycellulose, hydroxycellulose and similar substances.

Examples of isotonic agent include glucose, D-sorbitol, sodium chloride, glycerin, D-mannitol and similar substances.

Examples of buffers include buffer solutions such as phosphates, acetates, carbonates, citrates and similar substances.

Examples of a soothing agent include benzyl alcohol and similar substances.

Examples of preservatives include p-oxybenzoates, chlorobutanol, benzyl alcohol, phenylethyl alcohol, dehydroacetic acid, sorbic acid and similar substances.

Examples of antioxidants include sulfite, ascorbic acid, α-tocopherol and similar substances. The invention also includes a kit comprising a first pharmaceutical composition being as described above and at least an object for containing it such as a container, a divided bottle or a pack of divided parts. Typically, the kit includes instructions for administering the composition. The kit form is particularly advantageous when it comprises a further separate pharmaceutical composition comprising at least one further therapeutic agent, for example a chemotherapeutic agent, since it allows the separate compositions to be administered in different dosage forms (e.g., oral and parenteral) and/or different dosage ranges. An example of such a kit is the so-called blister. Blisters are well known in the packaging industry and are widely used for the packaging of dosage forms of pharmaceutical units (tablets, capsules and the like).

The dosage can vary widely and will, of course, be tailored to the individual needs in each particular case. In general, a daily dose can be from about 0.001 to 1000 mg/kg of body weight, preferably from about 0.1 to 20 mg/kg of body weight, preferably from about 0.5 to 4 mg/kg of body weight (for example about 300 mg per person), preferably divided into 1-3 single doses, which may consist, for example, in the same quantities. The compositions of the dosage units may contain submultiples thereof to compensate for the daily dose. The compound of the invention can be administered daily, weekly, or according to any other dosage regimen. The determination of the optimum dosages for a particular patient is well known to the experts of the sector. In particular, it will be clear that the upper and lower limits indicated here may be exceeded when this is indicated by the doctor, pharmacist, etc.

The synthetic schemes of Example 1 are examples of procedures for the preparation of a compound of formula (I). It should be understood that the compounds of the invention could be prepared by processes different from those illustrated in Example 1 and that the conversions depicted therein could be obtained using conditions different from those indicated. According to such synthetic schemes, it is useful to protect reactive functional groups to avoid unwanted reactions. Conventional protective groups can be used according to standard procedures, as described for example by T. Greene and P.G.M. Wuts in "Protective Groups in Organic Chemistry", John Wiley & Sons, 1991. Preferably, the hydroxyl groups are masked as alkoxy groups, in particular as methoxyl, benzyl or acetyl groups.

Furthermore, the technician of the sector will understand how to convert some compounds of the invention into further compounds of the invention through standard procedures, for example salification or desalting.

The present invention will be described by means of non-limiting examples, with reference to the following figures:

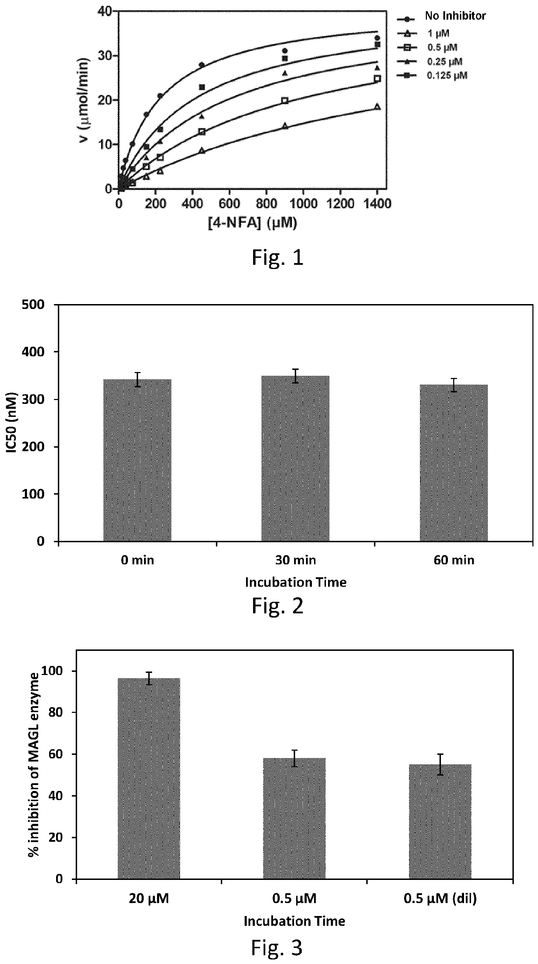

**Fig. 1****.** Inhibition of the activity of the MAGL enzyme and competitive inhibition of compound 15 (*K*ᵢ = 146 nM, α >10000). 4-NPA = 4-nitrophenyl acetate, v = enzymatic speed, α = analysis of the mechanism of action; when as in this case alpha is very large, the binding of the inhibitor prevents the binding of the substrate and there is competitive inhibition.
**Fig. 2****.** Inhibition values (IC₅₀, nM) of compound 15 at different incubation times with the MAGL enzyme (0 min, 30 min and 60 min).
**Fig. 3****.** Dilution assay: the first two columns indicate the percentage of inhibition of compound 15 at concentrations of 20 and 0.5 µM. The third column "0.5 µM (dil)" indicates the percentage of inhibition of compound 15 at a concentration of 20 µM after 40X dilution (final concentration = 0.5 µM).

### EXAMPLES

### Example 1: Synthesis of compounds

The compounds of the present invention (examples 1-17) can be prepared following the procedures described in the following schemes (schemes 1-4), specific for each series of examples. In the procedures described below, all temperatures are in degrees Celsius. The following abbreviations or reagents are explained as follows: room temperature, 20-25 °C (rt), hours (h), minutes (min.), potassium carbonate (K₂CO₃), sodium chloride (NaCI), dichloromethane (DCM) , ethyl acetate (EtOAc), triethylamine (EtsN), *N*,*N*-dimethylformamide (DMF), equivalents (eq), molar concentration (M), volume/volume ratio (v/v), millimoles (mmol), milliliters (mL), thin layer chromatography (TLC), nuclear magnetic resonance (NMR), palladium acetate (Pd(OAc)₂), triphenylphosphine (PPh₃), boron tribromide (BBr₃), 1-[bis (dimethylamino) methylene]-1*H*-1,2,3-triazole [4,5-*b*] pyridinium 3-oxide hexafluorophosphate (HATU), *N*,*N*-diisopropylethylamine (DIPEA), nuclear magnetic resonance (NMR), deuterated chloroform (CDCl₃), deuterated acetone (acetone-d₆), deuterated methanol (CD₃OD).

HPLC analysis: all final compounds assayed in biological tests are of a purity ≥ 95%, confirmed by HPLC with UV analysis (λ = 254 nm). Reverse phase analytical HPLC analysis was conducted using a Kinetex EVO C18 column (5 µm, 150 mm x 4.6 mm, Phenomenex, Inc.). Compounds **1-7**, **10-17** were analyzed using the "A" method: eluent A, water; eluent B, CH₃CN; after 5 min at 25% B, a gradient from 25% to 75% B is formed in 5 min and held at 75% B for 10 min; the flow rate is 1 mL/min. Compounds **8** and **9** (more lipophilic due to the long aliphatic chain) were analyzed using the "B" method: eluent A, water; eluent B, CH₃CN; after 4 min at 30% B, a gradient from 30% to 75% of B is formed in 3 min and held at 75% of B for 3 min; then a gradient from 75% to 95% of B is formed in 3 min and kept at 95% of B for 12 min; the flow rate is 1 mL/min.

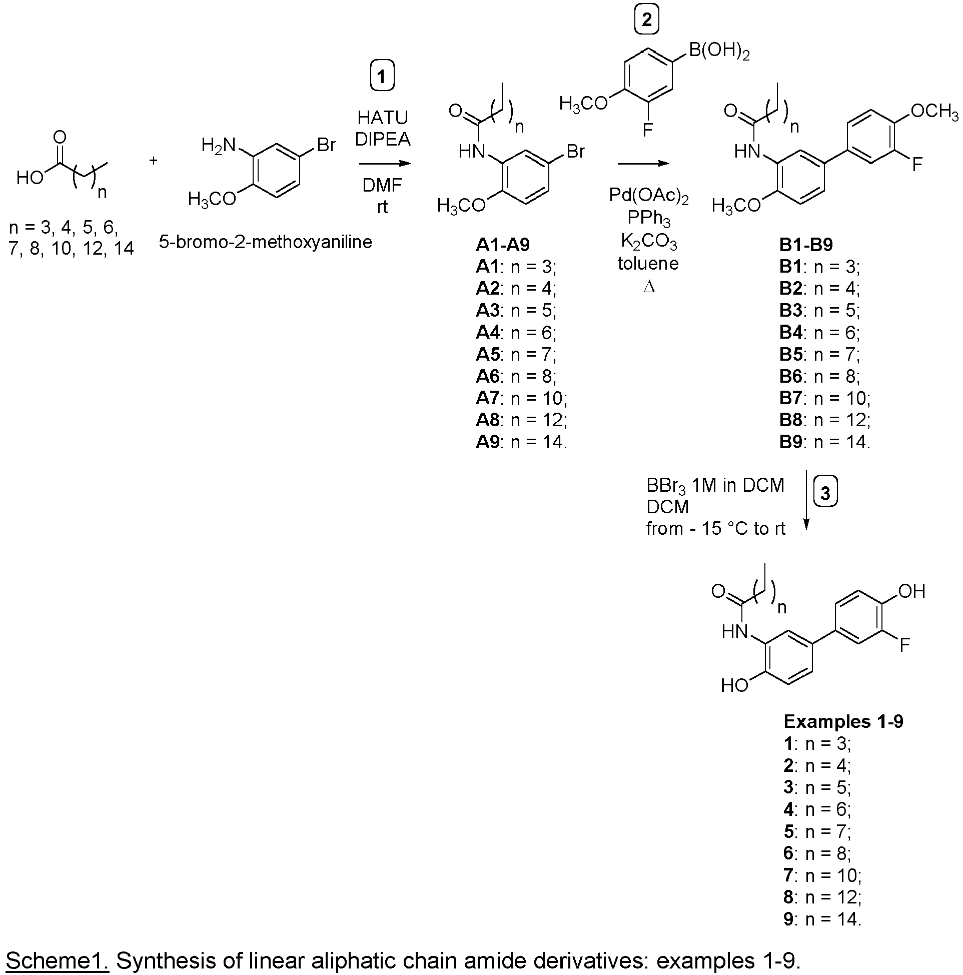

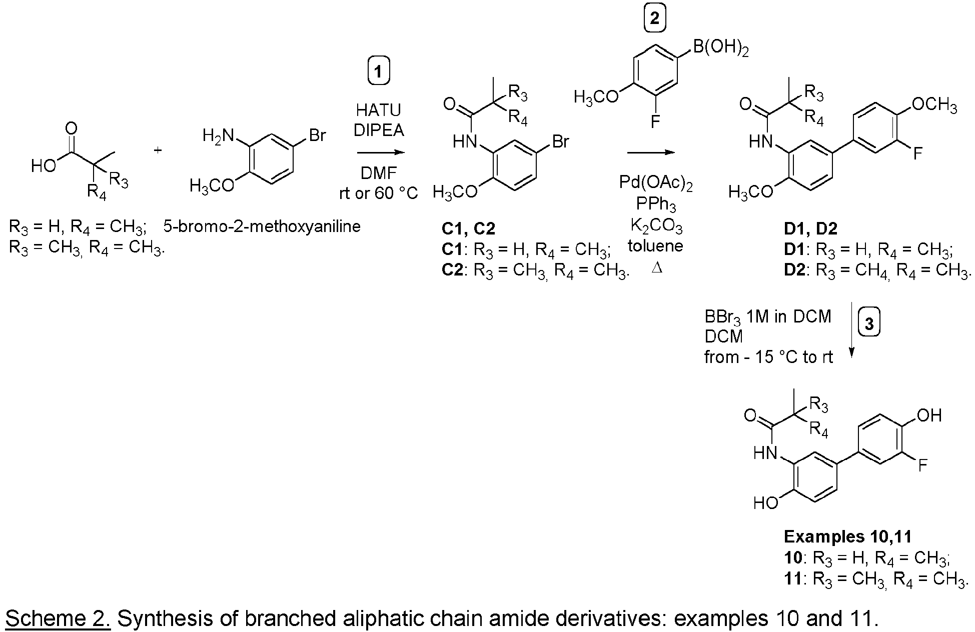

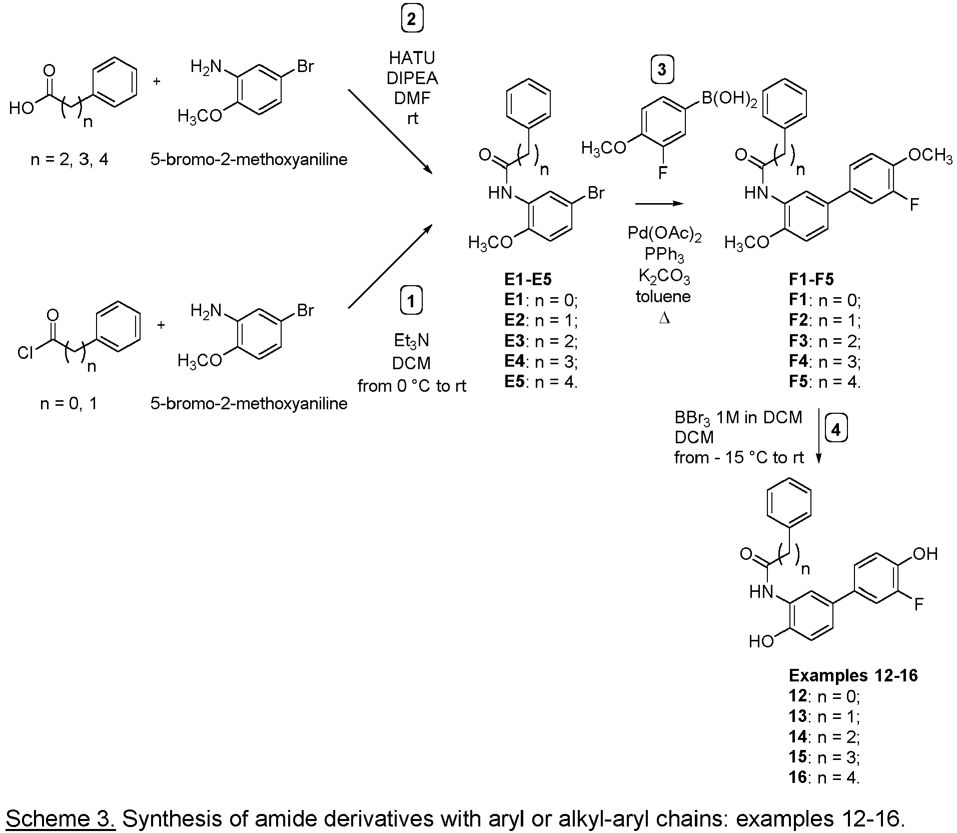

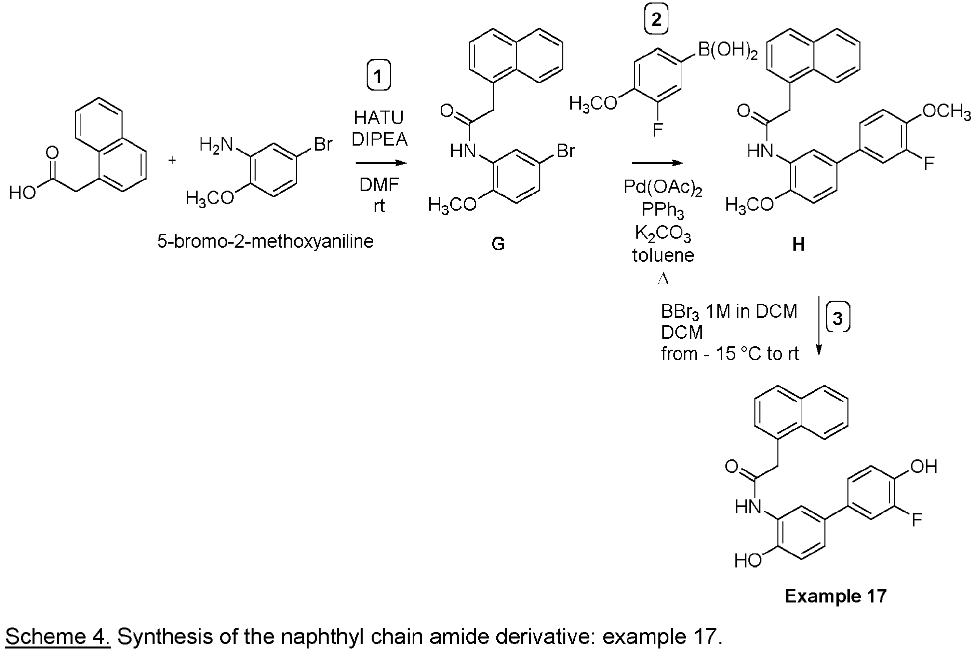

### Characterization of compounds 1-17

***N*-(3'-Fluoro-4,4'-dihydroxy-[1,1'-biphenyl]-3-yl)pentanamide (Example 1).** ¹H-NMR (acetone-*d*₆) δ (ppm): 0.94 (t, 3H, *J* = 7.4 Hz), 1.42 (sest, 2H, *J* = 7.5 Hz), 1.71 (quint, 2H, *J* = 7.5 Hz), 2.54 (t, 2H, *J* = 7.5 Hz), 6.95 (d, 1H, *J* = 8.4 Hz), 7.04 (dd, 1H, *J* = 9.1, 8.4 Hz), 7.23 (ddd, 1H, *J* = 8.4, 2.2, 1.0 Hz), 7.28 (d, 1H, *J* = 2.3 Hz), 7.30 (dd, 1H, *J* = 4.6, 2.3 Hz), 7.69 (d, 1H, *J* = 2.3 Hz), 9.23 (bs, 1H). ¹³C-NMR (acetone-*d*₆) δ (ppm): 14.08, 22.94, 28.54, 36.86, 114.58 (d, *J* = 19.1 Hz), 118.99 (d, *J* = 3.0 Hz), 119.30, 120.81, 123.18 (d, *J* = 2.9 Hz), 124.51, 128.00, 132.44 (d, *J* = 2.2 Hz), 133.78 (d, *J* = 6.2 Hz), 144.71 (d, *J* = 13.2 Hz), 148.70, 152.57 (d, *J* = 239.9 Hz), 174.36. HPLC analysis (method A): retention time = 11.400 min; peak area, 96% (254 nm).

***N*-(3'-Fluoro-4,4'-dihydroxy-[1,1'-biphenyl]-3-yl)hexanamide (Example 2).** ¹H-NMR (acetone-*d*₆) δ (ppm): 0.91 (t, 3H, *J* = 7.0 Hz), 1.32-1.45 (m, 4H), 1.73 (quint, 2H, *J* = 7.3 Hz), 2.53 (t, 2H, *J* = 7.5 Hz), 6.95 (d, 1H, *J* = 8.4 Hz), 7.04 (dd, 1H, *J* = 9.0, 8.5 Hz), 7.23 (ddd, 1H, *J* = 8.4, 2.2, 0.9 Hz), 7.28 (d, 1H, *J* = 2.2 Hz), 7.30 (dd, 1H, *J* = 4.3, 2.2 Hz), 7.69 (d, 1H, *J* = 2.2 Hz), 9.27 (bs, 1H). ¹³C-NMR (acetone-*d*₆) δ (ppm): 14.21, 23.07, 26.12, 32.08, 37.10, 114.58 (d, *J* = 19.3 Hz), 118.99 (d, *J* = 3.0 Hz), 119.32, 120.82, 123.18 (d, *J* = 3.5 Hz), 124.53, 128.00, 132.45 (d, *J* = 2.1 Hz), 133.80 (d, *J* = 6.1 Hz), 144.69 (d, *J* = 13.2 Hz), 148.70, 152.57 (d, *J* = 239.7 Hz), 174.37. HPLC analysis (method A): retention time = 11.967 min; peak area, 98% (254 nm).

***N*-(3'-Fluoro-4,4'-dihydroxy-[1,1'-biphenyl]-3-yl)eptanamide (Example 3).** ¹H-NMR (acetone-*d*₆) δ (ppm): 0.89 (t, 3H, *J* = 7.1 Hz), 1.25-1.47 (m, 6H),1.73 (quint, 2H, *J* = 7.5 Hz), 2.53 (t, 2H, *J* = 7.5 Hz), 6.95 (d, 1H, *J* = 8.4 Hz), 7.04 (dd, 1H, *J* = 9.2, 8.4 Hz), 7.22 (ddd, 1H, *J* = 8.3, 2.2, 1.0 Hz), 7.28 (d, 1H, *J* = 2.3 Hz), 7.30 (dd, 1H, *J =* 4.0, 2.3 Hz), 7.69 (d, 1H, *J* = 2.2 Hz), 9.22 (bs, 1H). ¹³C-NMR (acetone-*d*₆) δ (ppm): 14.30, 23.18, 26.40, 29.56, 32.29, 37.15, 114.59 (d, *J* = 19.1 Hz), 119.00 (d, *J* = 3.0 Hz), 119.33, 120.84, 123.18 (d, *J* = 2.9 Hz), 124.54, 128.00, 132.46 (d, *J* = 1.4 Hz), 133.81 (d, *J* = 6.0 Hz), 144.70 (d, *J* = 13.1 Hz), 148.72, 152.58 (d, *J* = 239.9 Hz), 174.40. HPLC analysis (method A): retention time = 12.491 min; peak area, 98% (254 nm).

***N*-(3'-Fluoro-4,4'-dihydroxy-[1,1'-biphenyl]-3-yl)octanamide (Example 4).** ¹H-NMR (acetone-*d*₆) δ (ppm): 0.88 (t, 3H, *J* = 7.0 Hz), 1.23-1.46 (m, 8H), 1.73 (quint, 2H, *J* = 7.5 Hz), 2.53 (t, 2H, *J* = 7.4 Hz), 6.95 (d, 1H, *J* = 8.4 Hz), 7.04 (dd, 1H, *J* = 9.1, 8.4 Hz), 7.23 (ddd, 1H, *J* = 8.4, 2.2, 1.0 Hz), 7.28 (d, 1H, *J* = 2.3 Hz), 7.30 (dd, 1H, *J =* 4.1, 2.3 Hz), 7.69 (d, 1H, *J =* 2.3 Hz), 9.24 (bs, 1H). ¹³C-NMR (acetone-*d*₆) δ (ppm): 23.24, 26.40, 29.22, 29.72, 30.38, 32.26, 37.09, 114.54 (d, *J* = 19.2 Hz), 118.95 (d, *J* = 3.4 Hz), 119.26, 120.77, 123.13 (d, *J =* 2.9 Hz), 124.47, 127.96, 132.40 (d, *J* = 1.5 Hz), 133.74 (d, *J* = 5.9 Hz), 144.68 (d, *J* = 13.1 Hz), 148.66, 152.53 (d, *J* = 239.9 Hz), 174.35. HPLC analysis (method A): retention time = 13.010 min; peak area, 96% (254 nm).

***N*-(3'-Fluoro-4,4'-dihydroxy-[1,1'-biphenyl]-3-yl)nonanamide (Example 5).** ¹H-NMR (acetone-*d*₆) δ (ppm): 0.88 (t, 3H, *J* = 7.0 Hz),1.25-1.45 (m, 10H), 1.73 (quint, 2H, *J* = 7.4 Hz), 2.53 (t, 2H, *J* = 7.5 Hz), 6.95 (d, 1H, *J* = 8.4 Hz), 7.04 (dd, 1H, *J* = 9.1, 8.4 Hz), 7.23 (ddd, 1H, *J* = 8.4, 2.2, 1.0 Hz), 7.28 (d, 1H, *J* = 2.3 Hz), 7.30 (dd, 1H, *J* = 3.8, 2.3 Hz), 7.69 (d, 1H, *J =* 2.3 Hz), 9.22 (bs, 1H). ¹³C-NMR (acetone-*d*₆) δ (ppm): 14.34, 23.30, 26.45, 29.90, 29.92, 30.09, 32.58, 37.15, 114.60 (d, *J* = 19.1 Hz), 119.01 (d, *J* = 3.0 Hz), 119.35, 120.84, 123.19 (d, *J* = 3.1 Hz), 124.55, 128.01, 132.47 (d, *J* = 1.5 Hz), 133.83 (d, *J* = 6.4 Hz), 144.71 (d, *J* = 13.2 Hz), 148.73, 152.59 (d, *J* = 239.9 Hz), 174.40. HPLC analysis (method A): retention time = 13.494 min; peak area, 98% (254 nm).

***N*-(3'-Fluoro-4,4'-dihydroxy-[1,1'-biphenyl]-3-yl)decanamide (Example 6).** ¹H-NMR (acetone-*d*₆) δ (ppm): 0.87 (t, 3H, *J* = 6.8 Hz), 1.20-1.46 (m, 12H), 1.73 (quint, 2H, *J* = 7.3 Hz), 2.53 (t, 2H, *J* = 7.5 Hz), 6.95 (d, 1H, *J* = 8.3 Hz), 7.04 (t, 1H, *J* = 8.8 Hz), 7.20-7.25 (m, 1H), 7.28 (d, 1H, *J* = 2.2 Hz), 7.30 (dd, 1H, *J* = 4.4, 2.2 Hz), 7.70 (d, 1H, *J* = 2.3 Hz), 9.25 (bs, 1H). ¹³C-NMR (acetone-*d*₆) δ (ppm): 14.35 (2C), 23.32, 26.44, 29.88, 30.09, 30.21, 32.60, 37.14, 114.57 (d, *J* = 19.1 Hz), 118.99 (d, *J* = 3.3 Hz), 119.28, 120.78, 123.17 (d, *J* = 3.1 Hz), 124.48, 128.02, 132.40 (d, *J* = 1.9 Hz), 133.76 (d, *J* = 6.3 Hz), 144.74 (d, *J* = 13.2 Hz), 148.72, 152.58 (d, *J* = 239.6 Hz), 174.37. HPLC analysis (method A): retention time = 14.027 min; peak area, 95% (254 nm).

***N*-(3'-Fluoro-4,4'-dihydroxy-[1,1'-biphenyl]-3-yl)dodecanamide (Example 7).** ¹H-NMR (acetone-*d*₆) δ (ppm): 0.87 (t, 3H, *J* = 6.9 Hz), 1.22-1.47 (m, 16H), 1.73 (quint, 2H, *J* = 7.4 Hz), 2.53 (t, 2H, *J* = 7.5 Hz), 6.95 (d, 1H, *J* = 8.3 Hz), 7.04 (t, 1H, *J* = 8.8 Hz), 7.23 (ddd, 1H, *J =* 8.4, 2.2, 0.9 Hz), 7.28 (d, 1H, *J* = 2.3 Hz), 7.30 (dd, 1H, *J* = 3.7, 2.3 Hz), 7.69 (d, 1H, *J* = 2.3 Hz), 9.23 (bs, 1H). ¹³C-NMR (acetone-*d*₆) δ (ppm): 14.34, 23.32, 26.44, 29.89, 30.06, 30.08, 30.24, 30.33, 30.35, 32.63, 37.15, 114.58 (d, *J* = 19.4 Hz), 119.00 (d, *J* = 3.2 Hz), 119.33, 120.83, 123.18 (d, *J* = 2.9 Hz), 124.53, 128.01, 132.45 (d, *J* = 1.5 Hz), 133.82 (d, *J* = 6.6 Hz), 144.71 (d, *J* = 13.2 Hz), 148.73, 152.59 (d, *J* = 239.9 Hz), 174.41. HPLC analysis (method A): retention time = 15.656 min; peak area, 99% (254 nm).

***N*-(3'-Fluoro-4,4'-dihydroxy-[1,1'-biphenyl]-3-yl)tetradecanamide (Example 8).** ¹H-NMR (acetone-*d*₆) δ (ppm): 0.87 (t, 3H, *J* = 6.9 Hz), 1.22-1.47 (m, 20H), 1.73 (quint, 2H, *J* = 7.3 Hz), 2.53 (t, 2H, *J* = 7.4 Hz), 6.95 (d, 1H, *J* = 8.4 Hz), 7.04 (dd, 1H, *J* = 9.1, 8.4 Hz), 7.23 (ddd, 1H, *J* = 8.4, 2.2, 1.0 Hz), 7.28 (d, 1H, *J* = 2.3 Hz), 7.30 (dd, 1H, *J* = 3.9, 2.3 Hz), 7.69 (d, 1H, *J =* 2.3 Hz), 9.23 (bs, 1H). ¹³C-NMR (acetone-*d*₆) δ (ppm): 14.35, 23.33 (2C), 26.44, 29.88, 30.07 (2C), 30.24, 30.35, 30.36, 30.37, 32.64, 37.14, 114.58 (d, *J* = 19.1 Hz), 118.99 (d, *J* = 4.0 Hz), 119.33, 120.82, 123.17 (d, *J* = 3.0 Hz), 124.53, 128.00, 132.44 (d, *J* = 2.0 Hz), 133.80 (d, *J* = 6.0 Hz), 144.71 (d, *J* = 13.1 Hz), 148.72, 152.58 (d, *J* = 239.6 Hz), 174.40. HPLC analysis (method B): retention time = 15.083 min; peak area, 99% (254 nm).

***N*-(3'-Fluoro-4,4'-dihydroxy-[1,1'-biphenyl]-3-yl)palmitamide (Example 9).** ¹H-NMR (acetone-*d*₆) δ (ppm): 0.87 (t, 3H, *J* = 6.8 Hz), 1.20-1.46 (m, 24H), 1.73 (quint, 2H, *J* = 7.3 Hz), 2.53 (t, 2H, *J* = 7.4 Hz), 6.95 (d, 1H, *J* = 8.4 Hz), 7.04 (dd, 1H, *J* = 9.1, 8.5 Hz), 7.22 (ddd, 1H, *J* = 8.4, 2.3, 0.9 Hz), 7.28 (d, 1H, *J* = 2.3 Hz), 7.30 (dd, 1H, *J* = 3.9, 2.3 Hz), 7.69 (d, 1H, *J =* 2.2 Hz), 9.23 (bs, 1H). ¹³C-NMR (acetone-*d*₆) δ (ppm): 14.35 (2C), 23.32, 26.44, 29.88, 30.08 (2C), 30.24 (2C), 30.34, 30.36, 30.37, 32.63 (2C), 37.13, 114.57 (d, *J* = 19.1 Hz), 118.98 (d, *J* = 2.9 Hz), 119.32, 120.80, 123.16 (d, *J* = 3.4 Hz), 124.52, 127.99, 132.43 (d, *J* = 2.2 Hz), 133.78 (d, *J* = 6.5 Hz), 144.69 (d, *J* = 13.2 Hz), 148.70, 152.56 (d, *J* = 239.6 Hz), 174.39. HPLC analysis (method B): retention time = 16.578 min; peak area, 98% (254 nm).

***N*-(3'-Fluoro-4,4'-dihydroxy-[1,1'-biphenyl]-3-yl)isobutyramide (Example 10).** ¹H-NMR (acetone-*d*₆) δ (ppm): 1.23 (d, 6H, *J* = 6.8 Hz), 2.78-2.92 (m, 1H), 6.95 (d, 1H, *J* = 8.4 Hz), 7.04 (dd, 1H, *J* = 9.1, 8.4 Hz), 7.23 (ddd, 1H, *J =* 8.4, 2.2, 1.0 Hz), 7.28 (dd, 1H, *J* = 2.2, 1.2 Hz), 7.31 (dd, 1H, *J* = 5.4, 2.3 Hz), 7.73 (d, 1H, *J* = 2.3 Hz), 9.19 (bs, 1H). ¹³C-NMR (acetone-*d*₆) δ (ppm): 19.97 (2C), 36.28, 114.56 (d, *J* = 19.1 Hz), 118.97 (d, *J* = 2.9 Hz), 119.24, 120.86, 123.16 (d, *J* = 2.9 Hz), 124.49, 127.97, 132.41 (d, J = 1.4 Hz), 133.77 (d, *J* = 6.4 Hz), 144.68 (d, *J=* 13.2 Hz), 148.67, 152.55 (d, *J* = 239.9 Hz), 178.20. HPLC analysis (method A): retention time = 10.570 min; peak area, 100% (254 nm).

***N*-(3'-Fluoro-4,4'-dihydroxy-[1,1'-biphenyl]-3-yl)pivalamide (Example 11).** ¹H-NMR (acetone-*d*₆) δ (ppm): 1.35 (s, 9H), 6.97 (d, 1H, *J* = 8.4 Hz), 7.04 (dd, 1H, *J* = 9.2, 8.4 Hz), 7.24 (ddd, 1H, *J* = 8.4, 2.2, 1.0 Hz), 7.28 (dd, 1H, *J* = 8.4, 2.3 Hz), 7.31 (dd, 1H, *J* = 12.6, 2.2 Hz), 7.94 (d, 1H, *J* = 2.3 Hz), 8.72 (bs, 1H), 8.99 (bs, 1H). ¹³C-NMR (acetone-*d*₆) δ (ppm): 23.28, 27.83 (3C), 114.58 (d, *J* = 19.1 Hz), 118.53, 118.97 (d, *J* = 3.5 Hz), 120.84, 123.19 (d, *J* = 3.2 Hz), 124.11, 128.05, 132.47 (d, *J = 2.2* Hz), 133.87 (d, *J* = 6.3 Hz), 144.68 (d, *J =* 13.2 Hz), 148.40, 152.56 (d, *J* = 239.3 Hz), 179.05. HPLC analysis (method A): retention time = 11.326 min; peak area, 99% (254 nm).

***N*-(3'-Fluoro-4,4'-dihydroxy-[1,1'-biphenyl]-3-yl)benzamide (Example 12).** ¹H-NMR (acetone-*d*₆) δ (ppm): 7.04 (d, 1H, *J* = 8.5 Hz), 7.06 (dd, 1H, *J* = 9.1, 8.5 Hz), 7.29 (ddd, 1H, *J* = 8.4, 2.2, 1.0 Hz), 7.34 (dd, 1H, *J* = 3.3, 2.3 Hz), 7.37 (dd, 1H, *J* = 2.2, 0.8 Hz), 7.54-7.61 (m, 2H), 7.65 (tt, 1H, *J* = 7.3, 1.7 Hz), 8.02-8.11 (m, 3H), 9.03 (bs, 1H), 9.54 (bs, 1H). ¹³C-NMR (acetone-*d*₆) δ (ppm): 114.61 (d, *J* = 19.1 Hz), 118.84, 118.99 (d, *J* = 2.9 Hz), 121.45, 123.21 (d, *J* = 3.6 Hz), 124.73, 127.81, 128.46 (2C), 129.54 (2C), 132.61 (d, *J* = 1.4 Hz), 132.96, 133.78 (d, *J* = 6.0 Hz), 134.89, 144.71 (d, *J* = 13.2 Hz), 148.86, 152.57 (d, *J* = 239.9 Hz), 167.37. HPLC analysis (method A): retention time = 11.341 min; peak area, 99% (254 nm).

***N*-(3'-Fluoro-4,4'-dihydroxy-[1,1'-biphenyl]-3-yl)-2-phenylacetamide (Example 13).** ¹H-NMR (acetone-*d*₆) δ (ppm): 3.87 (s, 2H), 6.94 (d, 1H, *J* = 8.4 Hz), 7.03 (dd, 1H, *J* = 9.1, 8.5 Hz), 7.22 (ddd, 1H, *J* = 8.4, 2.2, 1.0 Hz), 7.24-7.32 (m, 3H), 7.32-7.39 (m, 2H), 7.40-7.47 (m, 2H), 7.91 (d, 1H, *J* = 2.2 Hz), 8.98 (bs, 1H),9.19 (bs, 1H). ¹³C-NMR (acetone-*d*₆) δ (ppm): 44.17, 114.59 (d, *J* = 19.1 Hz), 118.49, 118.97 (d, *J* = 2.9 Hz), 120.45, 123.21 (d, *J* = 3.0 Hz), 124.20, 127.78, 127.97, 129.40 (2C), 130.20 (2C), 132.52 (d, *J =* 1.4 Hz), 133.88 (d, *J =* 5.9 Hz), 136.41, 144.67 (d, *J* = 13.1 Hz), 148.10, 152.55 (d, *J* = 239.9 Hz), 171.63. HPLC analysis (method A): retention time = 11.356 min; peak area, 99% (254 nm).

***N*-(3'-Fluoro-4,4'-dihydroxy-[1,1'-biphenyl]-3-yl)-3-phenylpropanamide (Example 14).** ¹H-NMR (acetone-*d*₆) δ (ppm): 2.86 (t, 2H, *J* = 7.5 Hz), 3.05 (t, 2H, *J* = 7.7 Hz), 6.95 (d, 1H, *J =* 8.4 Hz), 7.04 (dd, 1H, *J* = 9.1, 8.5 Hz), 7.16-7.20 (m, 1H), 7.22 (ddd, 1H, *J* = 8.4, 2.2, 1.0 Hz), 7.25-7.32 (m, 6H), 7.70 (d, 1H, *J* = 2.3 Hz), 9.08 (bs, 1H), 9.20 (bs, 1H). ¹³C-NMR (acetone-*d₆*) δ (ppm): 32.22, 38.90, 114.58 (d, *J* = 19.1 Hz), 119.00 (d, *J* = 3.5 Hz), 119.10, 120.80, 123.18 (d, *J* = 3.3 Hz), 124.49, 126.97, 127.89, 129.26 (2C), 129.29 (2C), 132.45 (d, *J* = 1.6 Hz), 133.80 (d, *J* = 6.5 Hz), 141.91, 144.70 (d, *J* = 13.2 Hz), 148.59, 152.57 (d, *J* = 239.8 Hz), 173.33. HPLC analysis (method A): retention time = 11.817 min; peak area, 99% (254 nm).

***N*-(3'-Fluoro-4,4'-dihydroxy-[1,1'-biphenyl]-3-yl)-4-phenylbutanamide (Example 15).** ¹H-NMR (CD₃OD) δ (ppm): 2.03 (quint, 2H, *J* = 7.5 Hz), 2.48 (t, 2H, *J* = 7.5 Hz), 2.72 (t, 2H, *J* = 7.6 Hz), 6.90 (d, 1H, *J* = 8.3 Hz), 6.93 (d, 1H, *J* = 9.0, 8.4 Hz), 7.13-7.30 (m, 8H), 7.84 (d, 1H, *J* = 2.3 Hz). ¹³C-NMR (acetone-*d*₆) δ (ppm): 28.13, 35.80, 36.46, 114.60 (d, *J* = 19.1 Hz), 119.01 (d, *J* = 3.2 Hz), 119.25, 120.88, 123.19 (d, *J* = 2.9 Hz), 124.53, 126.74, 127.99, 129.22 (2C), 129.34 (2C), 132.47 (d, *J* = 1.8 Hz), 133.82 (d, *J* = 6.1 Hz), 142.69, 144.71 (d, *J* = 13.2 Hz), 148.69, 152.59 (d, *J* = 239.6 Hz), 174.01. HPLC analysis (method A): retention time = 12.218 min; peak area, 96% (254 nm).

***N*-(3'-Fluoro-4,4'-dihydroxy-[1,1'-biphenyl]-3-yl)-5-phenylpentanamide (Example 16).** ¹H-NMR (acetone-*d*₆) δ (ppm): 1.67-1.82 (m, 4H), 2.58 (t, 2H, *J* = 7.1 Hz), 2.67 (t, 2H, *J* = 7.3 Hz), 6.95 (d, 1H, *J* = 8.4 Hz), 7.04 (dd, 1H, *J* = 9.2, 8.4 Hz), 7.16 (tt, 1H, *J* = 7.0, 2.0 Hz), 7.19-7.32 (m, 7H), 7.70 (d, 1H, *J* = 2.3 Hz), 9.24 (bs, 1H). ¹³C-NMR (acetone-*d*₆) δ (ppm): 26.08, 31.84, 36.20, 36.96, 114.58 (d, *J* = 19.1 Hz), 118.99 (d, *J* = 3.3 Hz), 119.26, 120.81, 123.18 (d, *J* = 2.9 Hz), 124.51, 126.54 (2C), 127.98, 129.13 (2C), 129.23, 132.44 (d, *J* = 2.2 Hz), 133.78 (d, *J* = 6.2 Hz), 143.21, 144.70 (d, *J* = 13.2 Hz), 148.67, 152.57 (d, *J* = 239.9 Hz), 174.21. HPLC analysis (method A): retention time = 12.599 min; peak area, 99% (254 nm).

***N*-(3'-Fluoro-4,4'-dihydroxy-[1,1'-biphenyl]-3-yl)-2-(naphtalen-1-yl)acetamide (Example 17).** ¹H-NMR (acetone-*d*₆) δ (ppm): 4.36 (s, 2H), 6.90 (d, 1H, *J=* 8.4 Hz), 7.02 (dd, 1H, *J=* 9.1, 8.4 Hz), 7.17-7.24 (m, 2H), 7.27 (dd, 1H, *J* = 12.6, 2.2 Hz), 7.47-7.60 (m, 3H), 7.60-7.66 (m, 1H), 7.88 (d, 1H, *J* = 8.2 Hz), 7.92-7.97 (m, 1H), 7.99 (d, 1H, *J* = 2.2 Hz), 8.21 (d, 1H, *J* = 8.4 Hz), 9.13 (bs, 1H). ¹³C-NMR (acetone-*d*₆) δ (ppm): 41.97, 114.60 (d, *J* = 19.1 Hz), 118.14, 118.98 (d, *J* = 3.0 Hz), 120.18, 123.21 (d, *J* = 3.0 Hz), 123.95, 125.01, 126.50, 126.72, 127.21, 128.06, 128.81, 129.05, 129.51, 132.53 (d, *J* = 2.1 Hz), 132.72, 133.34, 133.94 (d, *J* = 6.5 Hz), 134.97, 144.67 (d, *J* = 12.8 Hz), 147.79, 152.56 (d, *J* = 239.9 Hz), 171.36. HPLC analysis (method A): retention time = 12.102 min; peak area, 99% (254 nm).

### Scheme 1.

### Step 1.

In a two-necked flask, the commercial carboxylic acid (1 eq) indicated below was dissolved in anhydrous DMF (3.4 ml) under Argon flow. Later HATU (1.05 eq) and DIPEA (4 eq) were added to this solution. After 30 min. by stirring at room temperature, commercial 5-bromo-2-methoxyaniline (150 mg, 1 eq, Merck-Sigma Aldrich, 750808-5G) was added to the mixture and the reaction was left under stirring at room temperature for about 3 h. The reaction mixture was then diluted with water and EtOAc, then the organic phase was washed repeatedly with a saturated solution of NaCl. The organic phase was dried over Na₂SO₄ and concentrated, to give a crude product which was purified by column chromatography on silica gel using mixtures of n-hexane/EtOAc or petroleum ether/EtOAc as eluent, to obtain the pure compounds A1 (yield 61%), A2 (yield 41%), A3 (yield 52%), A4 (yield 60%), A5 (yield 79%), A6 (yield 33%), A7 (yield 62%), A8 (yield 75%), A9 (yield 67%).

A1 was prepared starting from pentanoic acid (n = 3, Merck-Sigma Aldrich, 240370-100ML). A2 was prepared starting from hexanoic acid (n = 4, Merck-Sigma Aldrich, 153745-100G). A3 was prepared starting from heptanoic acid (n = 5, Merck-Sigma Aldrich, 75190-100ML). A4 was prepared starting from octanoic acid (n = 6, Merck-Sigma Aldrich, C2875-100ML). A5 was prepared starting from nonanoic acid (n = 7, Thermo Fisher Scientific-Alfa Aesar, B21568). A6 was prepared starting from decanoic acid (n = 8, Merck-Sigma Aldrich, C1875-100G). A7 was prepared starting from dodecanoic acid (n = 10, Merck-Sigma Aldrich, L556-25G). A8 was prepared starting from myristic acid (n = 12, Merck-Sigma Aldrich, 70082-50G). A9 was prepared starting from palmitic acid (n = 14, Merck-Sigma Aldrich, P5585-10G).

[A1: ¹H-NMR (CDCl₃) δ (ppm): 0.95 (t, 3H, *J* = 7.4 Hz), 1.41 (sext, 2H, *J* = 7.5 Hz), 1.71 (quint, 2H, *J* = 7.6 Hz), 2.39 (t, 2H, *J* = 7.5 Hz), 3.87 (s, 3H), 6.72 (d, 1H, *J* = 8.7 Hz), 7.13 (dd, 1H, *J* = 8.7, 2.4 Hz), 7.71 (bs, 1H), 8.61 (d, 1H, *J* = 2.3 Hz). A2: ¹H-NMR (CDCl₃) δ (ppm): 0.91 (t, 3H, *J* = 7.1 Hz), 1.32-1.40 (m, 4H), 1.73 (quint, 2H, *J* = 7.5 Hz), 2.38 (t, 2H, *J* = 7.5 Hz), 3.87 (s, 3H), 6.72 (d, 1H, *J* = 8.7 Hz), 7.13 (dd, 1H, *J* = 8.7, 2.4 Hz), 7.71 (bs, 1H), 8.61 (d, 1H, *J* = 2.1 Hz). A3: ¹H-NMR (CDCl₃) δ (ppm): 0.89 (t, 3H, *J* = 7.1 Hz), 1.23-1.43 (m, 6H),1.72 (quint, 2H, *J* = 7.5 Hz), 2.38 (t, 2H, *J* = 7.5 Hz), 3.87 (s, 3H), 6.72 (d, 1H, *J* = 8.7 Hz), 7.13 (dd, 1H, *J* = 8.6, 2.5 Hz), 7.70 (bs, 1H), 8.61 (d, 1H, *J* = 2.2 Hz). A4: ¹H-NMR (CDCl₃) δ (ppm): 0.88 (t, 3H, *J* = 7.1 Hz), 1.23-1.42 (m, 8H), 1.72 (quint, 2H, *J* = 7.4 Hz), 2.38 (t, 2H, *J* = 7.5 Hz), 3.87 (s, 3H), 6.72 (d, 1H, *J* = 8.7 Hz), 7.13 (dd, 1H, *J* = 8.7, 2.5 Hz), 7.70 (bs, 1H), 8.61 (d, 1H, *J* = 2.3 Hz). A5: ¹H-NMR (CDCl₃) δ (ppm): 0.88 (t, 3H, *J* = 7.0 Hz), 1.20-1.43 (m, 10H), 1.72 (quint, 2H, *J* = 7.4 Hz), 2.38 (t, 2H, *J* = 7.5 Hz), 3.87 (s, 3H), 6.72 (d, 1H, *J* = 8.6 Hz), 7.13 (dd, 1H, *J* = 8.6, 2.4 Hz), 7.70 (bs, 1H), 8.61 (d, 1H, *J = 2.2* Hz). A6: ¹H-NMR (CDCl₃) δ (ppm): 0.88 (t, 3H, *J* = 7.0 Hz), 1.22-1.41 (m, 12H), 1.72 (quint, 2H, *J* = 7.4 Hz), 2.38 (t, 2H, *J* = 7.5 Hz), 3.87 (s, 3H), 6.72 (d, 1H, *J* = 8.7 Hz), 7.13 (dd, 1H, *J* = 8.7, 2.4 Hz), 7.70 (bs, 1H), 8.61 (d, 1H, *J* = 2.3 Hz). A7: ¹H-NMR (CDCl₃) δ (ppm): 0.87 (t, 3H, *J* = 7.0 Hz), 1.20-1.45 (m, 16H), 1.71 (quint, 2H, *J* = 7.4 Hz), 2.38 (t, 2H, *J* = 7.5 Hz), 3.86 (s, 3H), 6.72 (d, 1H, *J* = 8.7 Hz), 7.13 (dd, 1H, *J* = 8.7, 2.4 Hz), 7.70 (bs, 1H), 8.61 (d, 1H, *J* = 2.2 Hz). A8: ¹H-NMR (CDCl₃) δ (ppm): 0.88 (t, 3H, *J* = 6.9 Hz), 1.17-1.45 (m, 20H), 1.71 (quint, 2H, *J* = 7.3 Hz), 2.38 (t, 2H, *J* = 7.5 Hz), 3.87 (s, 3H), 6.72 (d, 1H, *J* = 8.7 Hz), 7.13 (dd, 1H, *J* = 8.7, 2.4 Hz), 7.71 (bs, 1H), 8.61 (d, 1H, *J* = 2.2 Hz). A9: ¹H-NMR (CDCl₃) δ (ppm): 0.88 (t, 3H, *J* = 7.0 Hz), 1.15-1.42 (m, 24H), 1.72 (quint, 2H, *J* = 7.3 Hz), 2.38 (t, 2H, *J* = 7.6 Hz), 3.87 (s, 3H), 6.72 (d, 1H, *J* = 8.6 Hz), 7.14 (dd, 1H, *J* = 8.6, 2.5 Hz), 7.70 (bs, 1H), 8.61 (d, 1H, *J* = 2.1 Hz)].

### Step 2.

A solution of Pd(OAc)₂ (0.03 eq) and triphenylphosphine (0.15 eq) in anhydrous toluene (3.9 mL) was stirred under inert atmosphere at room temperature for 10 minutes. Subsequently, the intermediate A1, A2, A3, A4, A5, A6, A7, A8 or A9 (130 mg, 1 eq), anhydrous K₂CO₃ (1.5 eq), and the commercial 3-fluoro-4-methoxyphenylboronic acid (1.5 eq, Thermo Fisher Scientific-Alfa Aesar, L19818) were added. The resulting mixture was heated at 100 °C in a closed vial under an inert atmosphere overnight. After being cooled to room temperature, the mixture was diluted with water and extracted with EtOAc. The combined organic phases were washed with a saturated solution of NaCl, dried over anhydrous sodium sulphate and concentrated under vacuum. The crude residue was purified by flash chromatographic column, eluting with n-hexane/EtOAc or petroleum ether/EtOAc mixtures, to obtain the pure intermediates B1 (yield 63%), B2 (yield 70%), B3 (yield 51%), B4 (61% yield), B5 (26% yield), B6 (68% yield), B7 (50% yield), B8 (37% yield), B9 (46% yield).

B1 was prepared starting from intermediate A1, B2 was prepared starting from intermediate A2, B3 was prepared starting from intermediate A3, B4 was prepared starting from intermediate A4, B5 was prepared starting from intermediate A5, B6 was prepared from intermediate A6, B7 was prepared from intermediate A7, B8 was prepared from intermediate A8, B9 was prepared from intermediate A9.

[B1: ¹H-NMR (CDCl₃) δ (ppm): 0.96 (t, 3H, *J* = 7.3 Hz), 1.43 (sext, 2H, *J* = 7.4 Hz), 1.74 (quint, 2H, *J* = 7.6 Hz), 2.42 (t, 2H, *J* = 7.5 Hz), 3.91 (s, 3H), 3.92 (s, 3H), 6.91 (d, 1H, *J* = 8.5 Hz), 6.97 (t, 1H, *J* = 8.8 Hz), 7.19 (dd, 1H, *J* = 8.5, 2.3 Hz), 7.28-7.36 (m, 2H), 7.77 (bs, 1H),8.67 (d, 1H, *J* = 2.1 Hz). B2: ¹H-NMR (CDCl₃) δ (ppm): 0.92 (t, 3H, *J* = 7.0 Hz), 1.31-1.44 (m, 4H), 1.75 (quint, 2H, *J* = 7.2 Hz), 2.42 (t, 2H, *J* = 7.5 Hz), 3.91 (s, 3H), 3.92 (s, 3H), 6.91 (d, 1H, *J* = 8.5 Hz), 6.98 (t, 1H, *J* = 8.8 Hz), 7.19 (dd, 1H, *J* = 8.5, 2.2 Hz), 7.27-7.37 (m, 2H), 7.77 (bs, 1H), 8.68 (d, 1H, *J* = 2.1 Hz). B3: ¹H-NMR (CDCl₃) δ (ppm): 0.90 (t, 3H, *J* = 6.8 Hz), 1.30-1.47 (m, 6H), 1.75 (quint, 2H, *J* = 7.5 Hz), 2.42 (t, 2H, *J* = 7.5 Hz), 3.91 (s, 3H), 3.92 (s, 3H), 6.91 (d, 1H, *J* = 8.4 Hz), 6.98 (t, 1H, *J* = 8.8 Hz), 7.19 (dd, 1H, *J* = 8.4, 2.2 Hz), 7.28-7.37 (m, 2H), 7.77 (bs, 1H),8.68 (d, 1H, *J* = 1.6 Hz). B4: ¹H-NMR (CDCl₃) δ (ppm): 0.88 (t, 3H, *J* = 7.0 Hz), 1.20-1.44 (m, 8H), 1.75 (quint, 2H, *J* = 7.5 Hz), 2.41 (t, 2H, *J* = 7.5 Hz), 3.91 (s, 3H), 3.92 (s, 3H), 6.91 (d, 1H, *J* = 8.5 Hz), 6.97 (t, 1H, *J* = 8.8 Hz), 7.19 (dd, 1H, *J* = 8.4, 2.2 Hz), 7.28-7.36 (m, 2H), 7.78 (bs, 1H), 8.67 (d, 1H, *J* = 2.2 Hz). B5: ¹H-NMR (CDCl₃) δ (ppm): 0.88 (t, 3H, *J =* 6.9 Hz), 1.22-1.45 (m, 10H), 1.75 (quint, 2H, *J* = 7.5 Hz), 2.41 (t, 2H, *J* = 7.5 Hz), 3.91 (s, 3H), 3.92 (s, 3H), 6.91 (d, 1H, *J* = 8.4 Hz), 6.98 (t, 1H, *J* = 8.8 Hz), 7.19 (dd, 1H, *J* = 8.5, 2.2 Hz), 7.28-7.36 (m, 2H), 7.77 (bs, 1H), 8.68 (d, 1H, *J* = 2.0 Hz). B6: ¹H-NMR (CDCl₃) δ (ppm): 0.88 (t, 3H, *J* = 6.9 Hz), 1.19-1.45 (m, 12H), 1.75 (quint, 2H, *J* = 7.4 Hz), 2.42 (t, 2H, *J* = 7.5 Hz), 3.91 (s, 3H), 3.92 (s, 3H), 6.91 (d, 1H, *J* = 8.5 Hz), 6.97 (t, 1H, *J* = 8.8 Hz), 7.19 (dd, 1H, *J* = 8.4, 2.3 Hz), 7.29-7.36 (m, 2H), 7.78 (bs, 1H), 8.67 (d, 1H, *J* = 2.0 Hz). B7: ¹H-NMR (CDCl₃) δ (ppm): 0.88 (t, 3H, *J* = 7.0 Hz), 1.19-1.45 (m, 16H), 1.75 (quint, 2H, *J* = 7.5 Hz), 2.42 (t, 2H, *J* = 7.6 Hz), 3.91 (s, 3H), 3.92 (s, 3H), 6.92 (d, 1H, *J* = 8.5 Hz), 6.98 (t, 1H, *J* = 8.8 Hz), 7.19 (dd, 1H, *J* = 8.5, 2.2 Hz), 7.28-7.37 (m, 2H), 7.77 (bs, 1H), 8.68 (d, 1H, *J* = 2.2 Hz). B8: ¹H-NMR (CDCl₃) δ (ppm): 0.88 (t, 3H, *J* = 7.0 Hz), 1.18-1.44 (m, 20H), 1.74 (quint, 2H, *J* = 7.4 Hz), 2.41 (t, 2H, *J* = 7.5 Hz), 3.91 (s, 3H), 3.92 (s, 3H), 6.91 (d, 1H, *J* = 8.5 Hz), 6.97 (t, 1H, *J* = 8.8 Hz), 7.19 (dd, 1H, *J* = 8.5, 2.3 Hz), 7.28-7.36 (m, 2H), 7.77 (bs, 1H), 8.67 (d, 1H, *J* = 2.1 Hz). B9: ¹H-NMR (CDCl₃) δ (ppm): 0.88 (t, 3H, *J* = 7.0 Hz), 1.20-1.44 (m, 24H), 1.74 (quint, 2H, *J* = 7.4 Hz), 2.41 (t, 2H, *J* = 7.5 Hz), 3.91 (s, 3H), 3.92 (s, 3H), 6.91 (d, 1H, *J* = 8.4 Hz), 6.98 (t, 1H, *J* = 8.8 Hz), 7.19 (dd, 1H, *J* = 8.3, 2.3 Hz), 7.28-7.36 (m, 2H), 7.77 (bs, 1H), 8.67 (d, 1H, *J* = 1.9 Hz)].

### Step 3.

A solution of the methoxylated intermediate B1, B2, B3, B4, B5, B6, B7, B8 or B9 (0.200 mmol) in anhydrous DCM (2.3 mL) was cooled to -15 °C and treated dropwise with a solution 1 M of BBr₃ in dichloromethane (0.63 mL), and the resulting solution was left under stirring at 0 °C for 1 hour and at rt until the disappearance of the starting compound was verified by TLC analysis. The mixture was then diluted with water and extracted with ethyl acetate. The organic phase was washed with a saturated solution of NaCl, dried and evaporated. The residue was purified by silica gel chromatography (n-hexane/EtOAc mixtures) to obtain the pure phenolic compounds, examples 1-9 (example 1: 74% yield, example 2: 54% yield, example 3: 80% yield, example 4: yield 46%, example 5: yield 76%, example 6: yield 28%, example 7: yield 68%, example 8: yield 79%, example 9: yield 66%).

Example 1 was prepared starting from intermediate B1, Example 2 was prepared starting from intermediate B2, Example 3 was prepared starting from intermediate B3, Example 4 was prepared to starting from intermediate B4, Example 5 was prepared starting from intermediate B5, Example 6 was prepared starting from intermediate B6, Example 7 was prepared starting from intermediate B7, Example 8 was prepared starting from intermediate B8, Example 9 was prepared starting from intermediate B9.

### Scheme 2.

### Step 1.

In a two-necked flask, the commercial carboxylic acid (1 eq) indicated below was dissolved in anhydrous DMF (3.4 ml) under Argon flow. Later HATU (1.05 eq) and DIPEA (4 eq) were added to this solution. After 30 min. by stirring at room temperature, commercial 5-bromo-2-methoxyaniline (150 mg, 1 eq) was added to the mixture and the reaction was left under stirring at room temperature for about 3 h (in the case of compound C1) or at 60 °C overnight (in the case of compound C2). The reaction mixture was then diluted with water and EtOAc, then the organic phase was washed repeatedly with a saturated solution of NaCl. The organic phase was dried over Na₂SO₄ and concentrated, to give a crude product which was purified by column chromatography on silica gel using mixtures of *n*-hexane/EtOAc or petroleum ether/EtOAc as eluent, to obtain the pure compounds C1 (yield 45%) or C2 (yield 54%).

C1 was prepared starting from 2-methylpropionic acid (Merck-Sigma Aldrich, I1754-100ML). C2 was prepared starting from 2,2-dimethylpropionic acid (Merck-Sigma Aldrich, T71803-100ML).

[C1: ¹H-NMR (CDCl₃) δ (ppm): 1.26 (d, 6H, *J* = 6.9 Hz), 2.55 (sett, 1H, *J* = 6.9 Hz), 3.87 (s, 3H), 6.73 (d, 1H, *J* = 8.7 Hz), 7.13 (dd, 1H, *J* = 8.7, 2.4 Hz), 7.78 (bs, 1H), 8.63 (d, 1H, *J* = 2.4 Hz). C2: ¹H-NMR (CDCl₃) δ (ppm): 1.31 (s, 9H), 3.88 (s, 3H), 6.73 (d, 1H, *J* = 8.7 Hz), 7.13 (dd, 1H, *J* = 8.6, 2.4 Hz), 8.09 (bs, 1H), 8.64 (d, 1H, *J* = 2.4 Hz)].

### Step 2.

A solution of Pd(OAc)₂ (0.03 eq) and triphenylphosphine (0.15 eq) in anhydrous toluene (2.9 mL) was stirred under an inert atmosphere at room temperature for 10 minutes. Subsequently, the intermediate C1 or C2 (80 mg, 1 eq), anhydrous K₂CO₃ (1.5 eq), and the commercial 3-fluoro-4-methoxyphenylboronic acid (1.5 eq) were added in order. The resulting mixture was heated at 100 °C in a closed vial under an inert atmosphere overnight. After being cooled to room temperature, the mixture was diluted with water and extracted with EtOAc. The combined organic phases were washed with a saturated solution of NaCl, dried over anhydrous sodium sulphate and concentrated under vacuum. The crude residue was purified by flash chromatographic column, eluting with n-hexane/EtOAc or petroleum ether/EtOAc mixtures, to obtain the pure intermediates D1 (yield 76%) or D2 (yield 67%).

D1 was prepared starting from intermediate C1, D2 was prepared starting from intermediate C2.

[D1: ¹H-NMR (CDCl₃) δ (ppm): 1.28 (d, 6H, *J* = 6.9 Hz), 2.59 (sett, 1H, *J* = 6.9 Hz), 3.91 (s, 3H), 3.93 (s, 3H), 6.92 (d, 1H, *J* = 8.5 Hz), 6.97 (t, 1H, *J* = 8.7 Hz), 7.19 (dd, 1H, *J* = 8.4, 2.3 Hz), 7.28-7.37 (m, 2H), 7.85 (bs, 1H), 8.70 (d, 1H, *J* = 2.2 Hz). D2: ¹H-NMR (CDCl₃) δ (ppm): 1.34 (s, 9H), 3.91 (s, 3H), 3.93 (s, 3H), 6.92 (d, 1H, *J* = 8.5 Hz), 6.96 (t, 1H, *J* = 8.8 Hz), 7.19 (dd, 1H, *J* = 8.5, 2.3 Hz), 7.29-7.37 (m, 2H), 8.16 (bs, 1H), 8.71 (d, 1H, *J* = 2.3 Hz)].

### Step 3.

A solution of the methoxylated intermediate D1 or D2 (0.200 mmol) in anhydrous DCM (2.3 mL) was cooled to -15 °C and treated dropwise with a 1 M solution of BBr₃ in dichloromethane (0.63 mL), and the resulting solution was left under stirring at 0 °C for 1 hour and at rt until the disappearance of the starting compound was verified by TLC analysis. The mixture was then diluted with water and extracted with ethyl acetate. The organic phase was washed with a saturated solution of NaCl, dried and evaporated. The residue was purified by silica gel chromatography (*n*-hexane/EtOAc mixtures) to obtain the pure phenolic compounds, examples 10 and 11 (example 10: 39% yield, example 11: 73% yield).

Example 10 was prepared starting from intermediate D1, Example 11 was prepared starting from intermediate D2.

### Scheme 3.

### Step 1.

In a two-necked flask, under Argon flow, commercial 5-bromo-2-methoxyaniline (150 mg, 1 eq) is dissolved in anhydrous dichloromethane (1.5 mL), then Et₃N (1.2 eq) is added. The reaction mixture is cooled in an ice bath to 0 °C and commercial benzoyl chloride (for the synthesis of E1, Merck-Sigma Aldrich, 259950-100ML) or phenylacetyl chloride (for the synthesis of E2, Merck-Sigma Aldrich, P16753-100G) (1.1 eq) was added. After 90 minutes, the reaction mixture is diluted with water and repeatedly extracted with EtOAc, then the combined organic phase was washed with a saturated solution of NaCl. The organic phase was dried over Na₂SO₄ and concentrated, thus obtaining a crude product which was purified by column chromatography on silica gel using mixtures of n-hexane/EtOAc or petroleum ether/EtOAc as eluent, to obtain the pure compounds E1 (yield 94%) or E2 (yield 61%).

[E1: ¹H-NMR (CDCl₃) δ (ppm): 3.91 (s, 3H), 6.78 (d, 1H, *J* = 8.6 Hz), 7.12-7.23 (m, 1H), 7.46-7.65 (m, 3H), 7.88 (d, 2H, *J* = 7.9 Hz), 8.51 (bs, 1H), 8.73-8.80 (m, 1H). E2: ¹H-NMR (CDCl₃) δ (ppm): 3.70 (s, 3H), 3.75 (s, 2H), 6.65 (d, 1H, *J* = 8.7 Hz), 7.11 (dd, 1H, *J* = 8.7, 2.4 Hz), 7.30-7.37 (m, 3H), 7.37-7.44 (m, 2H), 7.75 (bs, 1H), 8.56 (d, 1H, *J* = 2.4 Hz)].

### Step 2.

In a two-necked flask, the commercial carboxylic acid (1 eq) indicated below was dissolved in anhydrous DMF (3.4 ml) under Argon flow. Later HATU (1.05 eq) and DIPEA (4 eq) were added to this solution. After 30 min. by stirring at room temperature, commercial 5-bromo-2-methoxyaniline (150 mg, 1 eq) was added to the mixture and the reaction was left under stirring at room temperature until the starting compounds disappeared (TLC). The reaction mixture was then diluted with water and EtOAc, then the organic phase was washed repeatedly with a saturated aqueous solution of NaCl. The organic phase was dried over Na₂SO₄ and concentrated, to give a crude which was purified by column chromatography on silica gel using mixtures of *n*-hexane/EtOAc or petroleum ether/EtOAc as eluent, to obtain the pure compounds E3 (yield 76%), E4 (yield 65%) or E5 (yield 73%).

E3 was prepared starting from 3-phenylpropionic acid (n = 2, Merck-Sigma Aldrich, 135232-5G). E4 was prepared starting from 4-phenylbutyric acid (n = 3, Merck-Sigma Aldrich, P21005-25G). E5 was prepared starting from 5-phenylpentanoic acid (n = 4, Merck-Sigma Aldrich, P37602-25G).

[E3: ¹H-NMR (CDCl₃) δ (ppm): 2.71 (t, 2H, *J* = 7.7 Hz), 3.06 (t, 2H, *J* = 7.7 Hz), 3.81 (s, 3H), 6.70 (d, 1H, *J* = 8.7 Hz), 7.13 (dd, 1H, *J* = 8.7, 2.4 Hz), 7.18-7.25 (m, 3H), 7.27-7.33 (m, 2H), 7.63 (bs, 1H), 8.60 (d, 1H, *J* = 2.2 Hz). E4: ¹H-NMR (CDCl₃) δ (ppm): 2.07 (quint, 2H, *J* = 7.5 Hz), 2.39 (t, 2H, *J* = 7.4 Hz), 2.72 (t, 2H, *J* = 7.4 Hz), 3.86 (s, 3H), 6.72 (d, 1H, *J* = 8.7 Hz), 7.14 (dd, 1H, *J* = 8.7, 2.4 Hz), 7.17-7.24 (m, 3H), 7.26- 7.33 (m, 2H), 7.67 (bs, 1H), 8.60 (d, 1H, *J=* 2.3 Hz). E5: ¹H-NMR (CDCl₃) δ (ppm): 1.65-1.85 (m, 4H), 2.40 (t, 2H, *J* = 7.2 Hz), 2.67 (t, 2H, *J* = 7.4 Hz), 3.86 (s, 3H), 6.72 (d, 1H, *J* = 8.7 Hz), 7.13 (dd, 1H, *J* = 8.7, 2.5 Hz), 7.15-7.21 (m, 3H), 7.26- 7.31 (m, 2H), 7.68 (bs, 1H), 8.60 (d, 1H, *J* = 2.3 Hz)].

### Step 3.

A solution of Pd(OAc)₂ (0.03 eq) and triphenylphosphine (0.15 eq) in anhydrous toluene (4.7 mL) was stirred under an inert atmosphere at room temperature for 10 minutes. Subsequently, the intermediate E1, E2, E3, E4 or E5 (169 mg, 1 eq), anhydrous K₂CO₃ (1.5 eq), and the commercial 3-fluoro-4-methoxyphenylboronic acid (1.5 eq) were added. The resulting mixture was heated at 100 °C in a closed vial under an inert atmosphere overnight. After being cooled to room temperature, the mixture was diluted with water and extracted with EtOAc. The combined organic phases were washed with a saturated aqueous solution of NaCl, dried over anhydrous sodium sulphate and concentrated under vacuum. The crude residue was purified by flash column chromatography, eluting with n-hexane/EtOAc or petroleum ether/EtOAc mixtures, to obtain the pure intermediates F1 (yield 69%), F2 (yield 60%), F3 (yield 71%), F4 (64% yield) or F5 (71% yield).

F1 was prepared starting from intermediate E1, F2 was prepared starting from intermediate E2, F3 was prepared starting from intermediate E3, F4 was prepared starting from intermediate E4, F5 was prepared starting from intermediate E5.

[F1: ¹H-NMR (CDCl₃) δ (ppm): 3.93 (s, 3H), 3.97 (s, 3H), 6.94-7.05 (m, 2H), 7.24 (d, 1H, *J* = 2.3 Hz), 7.33-7.41 (m, 2H), 7.48-7.61 (m, 3H), 7.90-7.95 (m, 2H), 8.59 (bs, 1H), 8.82 (d, 1H, *J* = 2.2 Hz). F2: ¹H-NMR (CDCl₃) δ (ppm): 3.77 (s, 3H), 3.78 (s, 2H), 3.91 (s, 3H), 6.85 (d, 1H, *J* = 8.5 Hz), 6.97 (t, 1H, *J* = 8.8 Hz), 7.17 (dd, 1H, *J* = 8.4, 2.3 Hz), 7.26-7.43 (m, 7H), 7.83 (bs, 1H), 8.62 (d, 1H, *J* = 2.2 Hz). F3: ¹H-NMR (CDCl₃) δ (ppm): 2.74 (t, 2H, *J* = 7.8 Hz), 3.09 (t, 2H, *J* = 7.7 Hz), 3.87 (s, 3H), 3.92 (s, 3H), 6.89 (d, 1H, *J* = 8.5 Hz), 6.99 (t, 1H, *J* = 8.8 Hz), 7.19 (dd, 1H, *J* = 8.4, 2.3 Hz), 7.21-7.24 (m, 2H), 7.28-7.36 (m, 5H), 7.71 (bs, 1H), 8.66 (d, 1H, *J* = 2.2 Hz). F4: ¹H-NMR (CDCl₃) δ (ppm): 2.10 (quint, 2H, *J* = 7.4 Hz), 2. 42 (t, 2H, *J* = 7.4 Hz), 2.74 (t, 2H, *J* = 7.5 Hz), 3.91 (s, 3H), 3.92 (s, 3H), 6.91 (d, 1H, *J* = 8.5 Hz), 6.98 (t, 1H, *J* = 8.8 Hz), 7.17-7.25 (m, 4H), 7.27-7.36 (m, 4H), 7.75 (bs, 1H), 8.67 (d, 1H, *J* = 2.1 Hz). F5: ¹H-NMR (CDCl₃) δ (ppm): 1.67-1.86 (m, 4H), 2.44 (t, 2H, *J* = 7.2 Hz), 2.68 (t, 2H, *J* = 7.4 Hz), 3.91 (s, 3H), 3.92 (s, 3H), 6.91 (d, 1H, *J* = 8.5 Hz), 6.98 (t, 1H, *J* = 8.7 Hz), 7.15-7.22 (m, 4H), 7.27-7.36 (m, 4H), 7.76 (bs, 1H), 8.66 (d, 1H, *J* = 2.2 Hz)].

### Step 4.

A solution of the methoxylated intermediate F1, F2, F3, F4 or F5 (0.256 mmol) in anhydrous DCM (3.0 mL) was cooled to -15 °C and treated dropwise with a 1 M solution of BBr₃ in dichloromethane (0.81 mL), and the resulting solution was left under stirring at 0 ° C for 1 hour and at rt until the disappearance of the starting compound was verified by TLC analysis. The mixture was then diluted with water and extracted with ethyl acetate. The organic phase was washed with a saturated solution of NaCl, dried and evaporated. The residue was purified by silica gel chromatography (*n*-hexane/EtOAc mixtures) to obtain the pure phenolic compounds, examples 12-16 (example 12: 85% yield, example 13: 83% yield, example 14: 67% yield, example 15: 77% yield, example 16: 59% yield).

Example 12 was prepared starting from intermediate F1, Example 13 was prepared starting from intermediate F2, Example 14 was prepared starting from intermediate F3, Example 15 was prepared to starting with intermediate F4, Example 16 was prepared starting with intermediate F5.

### Scheme 4.

### Step 1.

In a two-necked flask, commercial 1-naphthylacetic acid (1 eq, Merck-Sigma Aldrich, 317918-100G) was dissolved in anhydrous DMF (3.4 ml) under Argon flow. Later HATU (1.05 eq) and DIPEA (4 eq) were added to this solution. After 30 min. by stirring at room temperature, commercial 5-bromo-2-methoxyaniline (150 mg, 1 eq) was added to the mixture and the reaction was left under stirring at room temperature for about 3 h. The reaction mixture was then diluted with water and EtOAc, then the organic phase was washed repeatedly with a saturated solution of NaCl. The organic phase was dried over Na₂SO₄ and concentrated, to give a crude product which was purified by column chromatography on silica gel using a mixture of n-hexane/EtOAc 9:1 v/v as eluent, to obtain the pure compound G (yield 38%).

[G: ¹H-NMR (CDCl₃) δ (ppm): 3.46 (s, 3H), 4.20 (s, 2H), 6.55 (d, 1H *J* = 8.6 Hz), 7.05 (dd, 1H, *J* = 8.7, 2.4 Hz), 7.47-7.59 (m, 4H), 7.72 (bs, 1H), 7.84-7.94 (m, 2H), 7.99-8.06 (m, 1H), 8.52 (d, 1H, *J* = 2.4 Hz)].

### Step 2.

A solution of Pd(OAc)₂ (0.03 eq) and triphenylphosphine (0.15 eq) in anhydrous toluene (2.7 mL) was stirred under an inert atmosphere at room temperature for 10 minutes. Subsequently, the intermediate G (104 mg, 1 eq), anhydrous K₂CO₃ (1.5 eq), and the commercial 3-fluoro-4-methoxyphenylboronic acid (1.5 eq) were added in order. The resulting mixture was heated at 100 °C in a closed vial under an inert atmosphere overnight. After being cooled to room temperature, the mixture was diluted with water and extracted with EtOAc. The combined organic phases were washed with a saturated aqueous solution of NaCl, dried over anhydrous sodium sulphate and concentrated under vacuum. The crude residue was purified by flash column chromatography, eluting with 8:2 v/v n-hexane/EtOAc mixture, to obtain the pure intermediate H (yield 55%).

[H: ¹H-NMR (CDCl₃) δ (ppm): 3.53 (s, 3H), 3.90 (s, 3H), 4.23 (s, 2H), 6.74 (d, 1H, *J* = 8.4 Hz), 6.96 (t, 1H, *J* = 8.8 Hz), 7.11 (dd, 1H, *J* = 8.5, 2.3 Hz), 7.26-7.33 (m, 2H), 7.48-7.59 (m, 4H), 7.79 (bs, 1H), 7.84-7.94 (m, 2H), 8.01-8.10 (m, 1H), 8.57 (d, 1H, *J* = 2.2 Hz)].

### Step 3.

A solution of the methoxylated intermediate H (0.134 mmol) in anhydrous DCM (1.6 mL) was cooled to -15 °C and treated dropwise with a 1 M solution of BBr₃ in dichloromethane (0.42 mL), and the resulting solution was left under stirring at 0 ° C for 1 hour and at rt until the disappearance of the starting compound was verified by TLC analysis. The mixture was then diluted with water and extracted with ethyl acetate. The organic phase was washed with a saturated solution of NaCl, dried and evaporated. The residue was purified by silica gel chromatography (*n*-hexane/EtOAc 7:3 v/v mixture) to obtain the pure phenolic compound, example 17 (yield 77%).

### Example 2: In vitro screening to evaluate the activity on the recombinant human MAGL enzyme.

The compound 4-nitrophenyl acetate (4-NPA Merck-Sigma Aldrich, 8430750025) was used as a substrate for the recombinant human MAGL enzyme (Cayman Chemical, 10007812). IC₅₀ values were generated in microplates containing 96 wells. The catalytic reaction was carried out at room temperature, at a final volume of 200 µL in 10 mM Tris buffer at pH 7.2 containing 1 mM EDTA and 0.1 mg/ml BSA. A total of 150 µL of 4-NPA 133.3 µM in ethanol was added to 10 µL of DMSO containing the appropriate amount of compound (500 - 0.00256 µM). The reaction began with the addition of 40 µL of the MAGL enzyme (11 ng/well). This dose is sufficient to keep the dosage linear for 30 minutes. After the reaction continued for 30 minutes, the absorbance values were then measured using a Victor X3 (PerkinElmer^{®}) microplate reader at a wavelength of 405 nm. Two other analyzes were also performed in parallel: one reaction without compounds and the second containing neither compound nor enzyme. IC₅₀ values were obtained from these experimental data (Table 1). To remove possible false positive results, a blank analysis was carried out for each compound concentration and the final absorbance results were obtained, subtracting the absorbance produced by the presence of all components except the MAGL enzyme under the same conditions. The IC₅₀ values were calculated from the experimental data (absorbance) through the construction of dose-response curves.

**Table 1. Inhibitory activity of compounds 1-17 against the recombinant human MAGL enzyme**

| **#** | **CMPD ID** | **Structure** | **MAGL IC₅₀ (µM)** |
|---|---|---|---|
| **1** | DSC-3 | 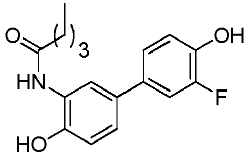 | 7.6 ± 0.1 |
| **2** | DSC-68-A | 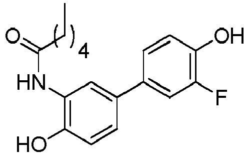 | 3.2 ± 0.2 |
| **3** | DSC-70 | 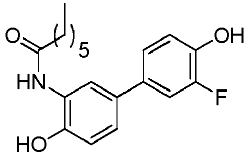 | 1.1 ± 0.1 |
| **4** | DSC-6 | 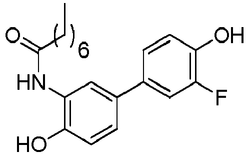 | 0.61 ± 0.03 |
| **5** | DSC-74 | 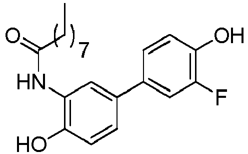 | 2.9 ± 0.1 |
| **6** | DSC-15-B | 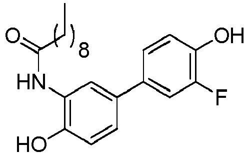 | 1.1 ± 0.1 |
| **7** | DSC-20 | 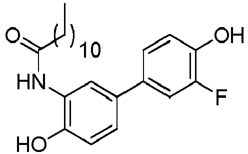 | 2.8 ± 0.3 |
| **8** | DSC-25 | 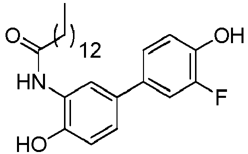 | 2.5 ± 0.4 |
| **9** | DSC-31 | 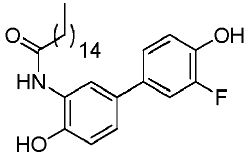 | > 20.0 |
| **10** | DSC-34-B | 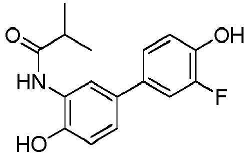 | 19.7 ± 1.0 |
| **11** | DSC-37 | 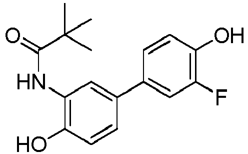 | > 20.0 |
| **12** | DSC-47 | 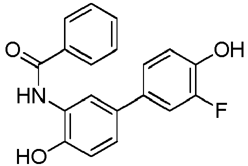 | 6.9 ± 0.4 |
| **13** | DSC-58 | 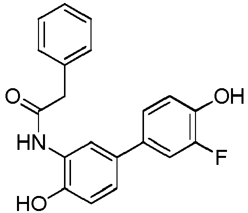 | 5.1 ± 0.5 |
| **14** | DSC-79-BIS | 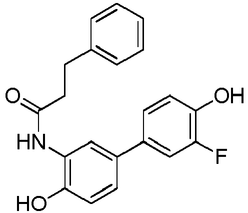 | 2.0 ± 0.1 |
| **15** | DSC-64 | 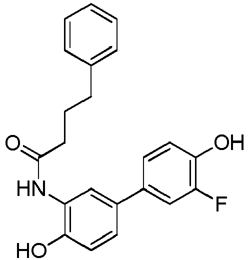 | 0.34 ± 0.01 |
| **16** | DSC-81-B | 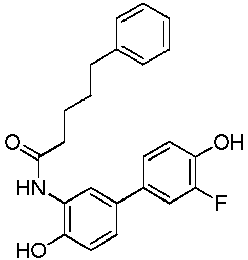 | 1.1 ± 0.1 |
| **17** | DSC-61 | 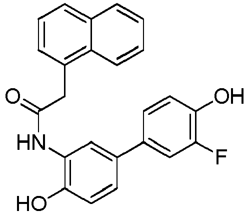 | 1.9 ± 0.1 |

All the compounds listed in Table 1 have been shown to be effective in inhibiting MAGL, with IC₅₀ in the nanomolar-micromolar order. The best results were obtained with derivatives **4** (IC₅₀ = 0.61 nM) and **15** (IC₅₀ = 0.34 nM). Compound 4 possess a linear 7-carbon alkyl chain. The amide chain of compound **4** has the optimal length for a correct interaction with MAGL. Similarly, compound **15,** with a phenyl-alkyl amide chain, in which the terminal phenyl is spaced by 3 carbon atoms from the carbonyl of the amide group, proved to be extremely potent.

In enzymatic kinetics experiments, compound 15 was tested in the presence of scalar concentrations of 4-NPA. It was added in staggered amounts (concentration range = 1-0.125 µM in DMSO) to a reaction mix containing staggered concentrations of 4-NPA (15-1400 µM in ethanol). Finally, the buffer solution containing the recombinant human MAGL enzyme (11 ng/well) was added. The enzymatic activity was measured at room temperature by recording the increase in absorbance corresponding to the increase in the concentration of 4-NPA using a Victor X3 microplate reader (PerkinElmer^{®}). The absorbance was converted into enzymatic rate by making appropriate use of positive and negative controls. The experimental data were analyzed by second order polynomial regression analysis applying the inhibition fit of the mixed model (Fig. 1).

As shown in Figure 1, the Michaelis-Menten type curve shows Km values of 0.230 ± 0.016 mM, Vmax = 41.3 ± 0.4 µmol • (min⁻¹mg⁻¹), a *K*ᵢ value for compound 15 of 146 ± 37 nM and an α value greater than 10000, thus supporting competitive behavior for the selected compound.

### Example 3: In vitro test to study the inhibition mechanism (reversible or irreversible)

Compound 15 was used as the reference compound for both tests.

### Pre-incubation assay with the recombinant human MAGL enzyme

The enzymatic reaction was carried out under the same conditions reported above. A total of 150 µL of solution containing the MAGL enzyme (11 ng/well) in buffer was added to 10 µL of DMSO containing the appropriate amount of compound 15 (40 - 0.00256 µM). After 0 minutes, 30 minutes and 60 minutes of incubation time, the reaction was started by adding 40 µL of 4-NPA 500 µM in buffer. The enzymatic activity was then measured according to the procedure described above. In principle, an irreversible inhibitor will increase its ability to block the enzyme with increasingly longer incubation times in the presence of the enzyme before adding the substrate; a constant IC₅₀ value, on the other hand, supports a reversible mechanism. As reported in Fig. 2, compound 15 did not show any significant increase in its ability to block the activity of the MAGL enzyme after 30 and 60 minutes, suggesting a reversible interaction with the enzyme.

### MAGL dilution assay with the recombinant human MAGL enzyme

The recombinant human MAGL enzyme (880 ng in 75 µl of Tris buffer, pH 7.2) was incubated for 60 minutes at room temperature with 5 µL of compound 15 (concentration of 12 µM and 0.3 µM in the mixture) dissolved in DMSO. The mixture containing the inhibitor and the enzyme was then diluted 40 times with the buffer solution. After 15 minutes of incubation, the reaction was started in a 160 µL aliquot with the addition of 40 µL of 4-NPA 500 µM in buffer and the enzyme activity was measured according to the procedure described above.

The % of inhibition of the MAGL enzyme was calculated from the measured absorbance. The absorbance measured in the presence of only enzyme with vehicle and without compound was considered as absence of inhibition (positive control), while the absorbance measured in the wells with only vehicle without enzyme and without compound was considered as 100% inhibition (negative control). Intermediate absorbance values with respect to these two extremes indicate enzymatic inhibition and are calculated as the difference between the absorbance value in the well with the compound and the absorbance value of the negative control, all scaled as a percentage considering as the maximum value the negative control and minimum value of minimum inhibition the positive control.

In dilution experiments, if compound 15 were an irreversible inhibitor, its inhibitory potency should not decrease upon dilution. Otherwise, inhibition levels should be substantially reduced following dilution in the presence of a reversible inhibitor. As shown in Fig. 3, compound 15 showed reversible inhibition, since the inhibition produced by a 20 µM concentration of the compound is significantly higher than the inhibition observed with a 40X dilution, which appears similar to that produced by a concentration of 0.5 µM of the compound.

### Example 4: Cell viability assays

The following tumor cell lines, MDA-MB-231 (Cell Biolabs AKR-231), HCT116 (ATCC^{®} CCL-247), CAOV3 (ATCC^{®} HTB-75), OVCAR3 (ATCC^{®} HTB-161) and SKOV3 (ATCC^{®} HTB-77) were kept at 37 °C in a humidified atmosphere containing 5% CO₂ according to the supplier. Cells (5 x 10²) were plated in 96-well culture plates. The day after plating, the compounds were dissolved in DMSO and added at different concentrations to the medium (final DMSO concentration < 5%). The compounds were added to the cell culture at a concentration between 200 and 0.02 µM. Cell viability was measured after 96 hours with the CellTiter-Glo^{®} Luminescent Cell Viability Assay kit in accordance with the supplier Promega (G7571) with a Tecan M1000 instrument. IC₅₀ values were calculated from dose-response curves. The means were obtained from the triplicates (Table 2).

**Table 2. Cell viability inhibition activity (IC₅₀, µM) of compound 15.**

| **IC₅₀ (µM, mean ± SD)** | | | | | |
|---|---|---|---|---|---|
| **Cpd** | **HCT116** | **MDA-MB-231** | **CAOV3** | **OVCAR3** | **SKOV3** |
| **15** | 14 ± 2 | 75 ± 6 | 34 ± 4 | 75 ± 4 | 16 ± 2 |

As shown in Table 2, compound 15 exhibits good cell viability inhibition activity for all cell lines tested.

## Claims

1. A compound of formula (I): 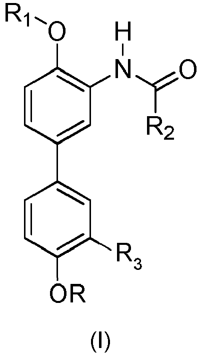 in which:
R and R₁ are independently selected from the group consisting of: H, linear C1-C6 alkyl chain, benzyl and acetyl;
R₂ is selected from the group consisting of: C1-C20 linear alkyl chain, C3-C10 branched alkyl chain, aryl, heteroaryl and C1-C10 linear alkyl chain substituted by aryl or heteroaryl;
R₃ is selected from the group consisting of: halogen, haloalkyl and CN;
the two rings shown are optionally and independently of each other replaced with one or more substituents independently selected from the group consisting of: halogen, linear or branched C1-C6 alkyl chain, haloalkyl, haloalkoxyl, CN, NO₂, NH₂, carboxylic ester, COOH and SH;
or a salt, stereoisomer, solvate or tautomer thereof.

2. The compound, salt, stereoisomer, solvate or tautomer according to claim 1, wherein R is H.

3. The compound, salt, stereoisomer, solvate or tautomer according to claim 1 or 2, wherein R₃ is selected from the group consisting of: F, Cl, Br, CF₃ and CN.

4. The compound, salt, stereoisomer, solvate or tautomer according to any one of previous claims, wherein R₃ is F.

5. The compound, salt, stereoisomer, solvate or tautomer according to any one of previous claims, wherein R is H and R₃ is F.

6. The compound, salt, stereoisomer, solvate or tautomer according to any one of previous claims, wherein R₁ is H.

7. The compound, salt, stereoisomer, solvate or tautomer according to any one of previous claims, wherein R₂ is selected from the group consisting of: n-butyl, n-pentyl, n-hexyl, n-heptyl, n-octyl, n -nonyl, n-undecanyl, n-tridecanyl, n-pentadecanyl, isopropyl, tert-butyl, phenyl, benzyl, (CH₂)₂phenyl, (CH₂)₃phenyl, (CH₂)₄phenyl and (CH₂)naphthyl.

8. The compound according to any one of previous claims being selected from the group consisting of:
| | | | | | |
|---|---|---|---|---|---|
| 1 | 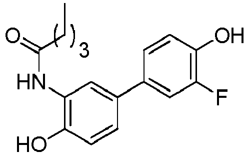 | | | 9 | 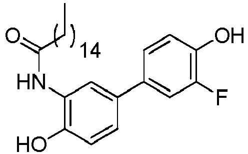 |
| 2 | 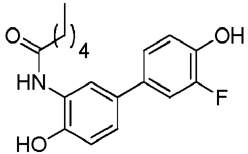 | | | 10 | 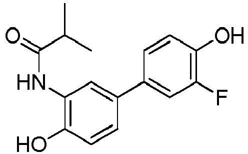 |
| 3 | 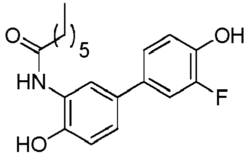 | | | 11 | 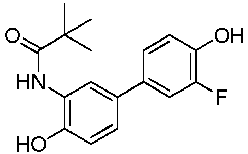 |
| 4 | 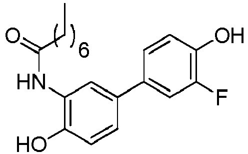 | | | 12 | 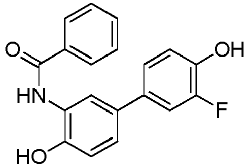 |
| 5 | 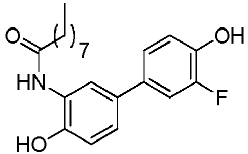 | | | 13 | 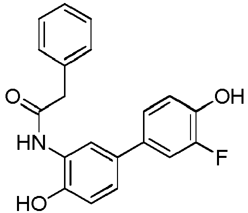 |
| 6 | 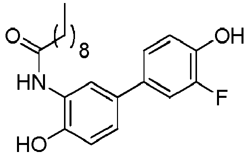 | | | 14 | 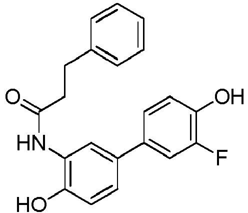 |
| 7 | 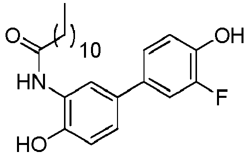 | | | 15 | 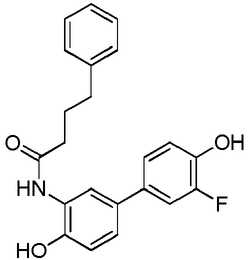 |
| 8 | 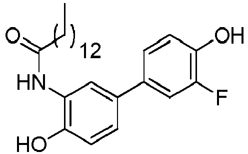 | | | 16 | 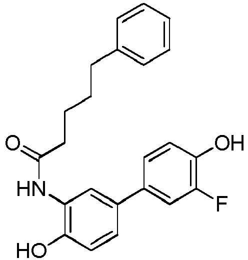 |
| | | 17 | 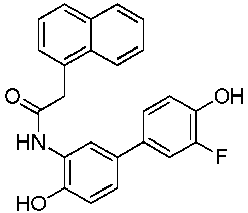 | | |
or a salt, stereoisomer, solvate or tautomer thereof.

9. The compound, salt, stereoisomer, solvate or tautomer as defined in any one of previous claims for use as a medicament.

10. The compound, salt, stereoisomer, solvate or tautomer as defined in any of previous claims for use as an inhibitor of the enzyme monoacylglycerol lipase (MAGL).

11. The compound, salt, stereoisomer, solvate or tautomer for use according to claim 10, wherein this inhibitor of MAGL enzyme is reversible.

12. The compound, salt, stereoisomer, solvate or tautomer as defined in any of previous claims for use in the prevention and/or treatment of a condition selected from the group consisting of: tumour, neuroinflammation, a neurodegenerative disease, pain, a neurological disorder, a psychiatric disorder, head injury, autoimmune disease, inflammation, abstinence, metabolic disorder, steatohepatitis, and any combination of thereof.

13. The compound, salt, stereoisomer, solvate or tautomer for use according to claim 12, wherein said tumor is selected from the group consisting of: colorectal cancer, breast cancer, ovarian cancer, pancreatic cancer, liver cancer and any combination thereof and/or wherein said neurodegenerative disease is selected from the group consisting of: Alzheimer's disease, Parkinson's disease, Huntington's disease, a demyelinating disease, such as multiple sclerosis, amyotrophic lateral sclerosis and acute disseminated encephalomyelitis, and any combination thereof and/or wherein said pain is acute or chronic and/or wherein said neurological disorder is selected from the group consisting of: epilepsy, migraine, neurotoxicity and stroke and/or wherein said psychiatric disorder is anxiety or depression and / or wherein said metabolic disorder is metabolic syndrome and/or wherein said steatohepatitis is non-alcoholic steatohepatitis.

14. A pharmaceutical composition comprising:
- the compound, salt, stereoisomer, solvate or tautomer as defined in any one of claims from 1 to 8,
- a pharmaceutically acceptable carrier, and
- optionally a further therapeutic agent.

15. The pharmaceutical composition as defined in claim 14 for use in the prevention and/or treatment of a condition as defined in claim 12 or 13.

16. A process for preparing a compound of formula (I), salt, stereoisomer, solvate or tautomer as defined in any one of claims from 1 to 8 wherein R and/or R₁ are H comprising the deprotection step of a compound of formula (VI): 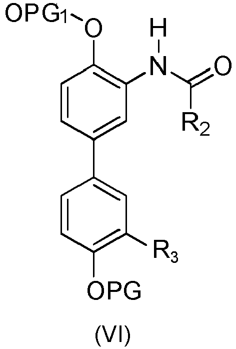 wherein PG and PG₁ are the same or different protecting groups.

## Patentansprüche

1. Verbindung der Formel (I): 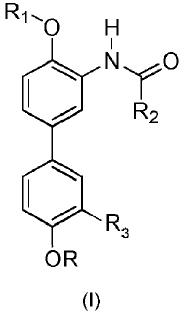 wobei:
R und R₁ unabhängig voneinander ausgewählt sind aus der Gruppe bestehend aus: H, linearer C1-C6-Alkylkette, Benzyl und Acetyl;
R₂ ausgewählt ist aus der Gruppe bestehend aus: linearer C1-C20-Alkylkette, verzweigter C3-C10-Alkylkette, Aryl, Heteroaryl und linearer C1-C10-Alkylkette, substituiert durch Aryl oder Heteroaryl; R₃ ausgewählt ist aus der Gruppe bestehend aus: Halogen, Halogenalkyl und CN;
die beiden gezeigten Ringe gegebenenfalls und unabhängig voneinander durch einen oder mehrere Substituenten ersetzt sind, die unabhängig voneinander ausgewählt sind aus der Gruppe bestehend aus: Halogen, linearer oder verzweigter C1-C6-Alkylkette, Halogenalkyl, Halogenalkoxyl, CN, NO₂ , NH₂, Carbonsäureester, COOH und SH;
oder ein Salz, Stereoisomer, Solvat oder Tautomer davon.

2. Verbindung, Salz, Stereoisomer, Solvat oder Tautomer nach Anspruch 1, wobei R für H steht.

3. Verbindung, Salz, Stereoisomer, Solvat oder Tautomer nach Anspruch 1 oder 2, wobei R₃ ausgewählt ist aus der Gruppe bestehend aus: F, Cl, Br, CF₃ und CN.

4. Verbindung, Salz, Stereoisomer, Solvat oder Tautomer nach einem der vorhergehenden Ansprüche, wobei R₃ für F steht.

5. Verbindung, Salz, Stereoisomer, Solvat oder Tautomer nach einem der vorhergehenden Ansprüche, wobei R für H steht und R₃ für F steht.

6. Verbindung, Salz, Stereoisomer, Solvat oder Tautomer nach einem der vorhergehenden Ansprüche, wobei R₁ für H steht.

7. Verbindung, Salz, Stereoisomer, Solvat oder Tautomer nach einem der vorhergehenden Ansprüche, wobei R₂ ausgewählt ist aus der Gruppe bestehend aus: n-Butyl, n-Pentyl, n-Hexyl, n-Heptyl, n-Octyl, n-Nonyl, n-Undecanyl, n-Tridecanyl, n-Pentadecanyl, Isopropyl, tert-Butyl, Phenyl, Benzyl, (CH₂)₂-Phenyl, (CH₂)₃-Phenyl, (CH₂)₄-Phenyl und (CH₂)Naphthyl.

8. Verbindung nach einem der vorhergehenden Ansprüche, ausgewählt aus der Gruppe bestehend aus:
| | | | |
|---|---|---|---|
| 1 | 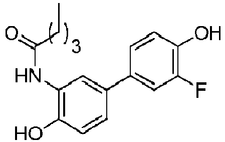 | 9 | 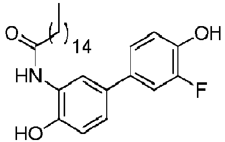 |
| 2 | 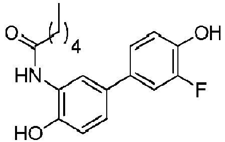 | 10 | 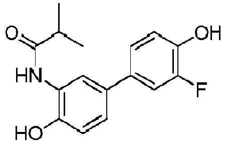 |
| 3 | 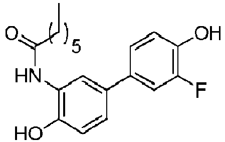 | 11 | 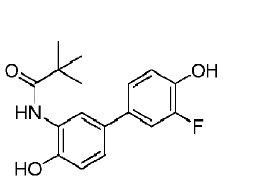 |
| 4 | 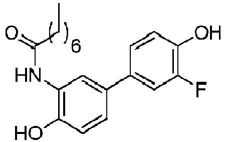 | 12 | 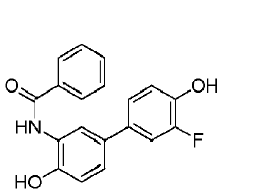 |
| 5 | 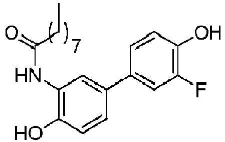 | 13 | 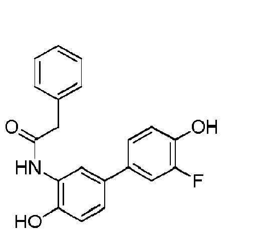 |
| 6 | 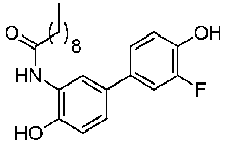 | 14 | 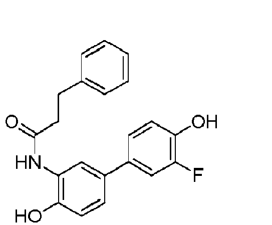 |
| 7 | 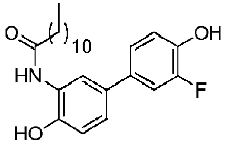 | 15 | 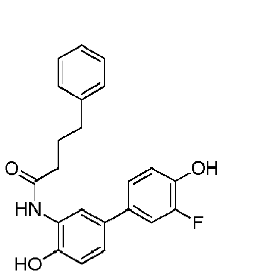 |
| 8 | 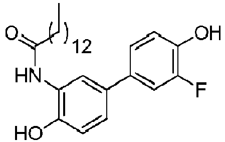 | 16 | 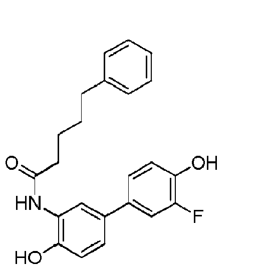 |
| 17 | 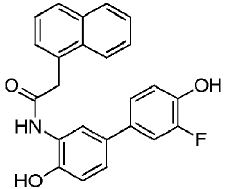 | | |
oder ein Salz, Stereoisomer, Solvat oder Tautomer davon.

9. Verbindung, Salz, Stereoisomer, Solvat oder Tautomer, wie in einem der vorhergehenden Ansprüche definiert, zur Verwendung als Medikament.

10. Verbindung, Salz, Stereoisomer, Solvat oder Tautomer, wie in einem der vorhergehenden Ansprüche definiert, zur Verwendung als Inhibitor des Enzyms Monoacylglycerinlipase (MAGL).

11. Verbindung, Salz, Stereoisomer, Solvat oder Tautomer zur Verwendung nach Anspruch 10, wobei dieser Inhibitor des MAGL-Enzyms reversibel ist.

12. Verbindung, Salz, Stereoisomer, Solvat oder Tautomer, wie in einem der vorhergehenden Ansprüche definiert, zur Verwendung bei der Vorbeugung und/oder Behandlung eines Zustands, ausgewählt aus der Gruppe bestehend aus: Tumor, Neuroinflammation, einer neurodegenerativen Erkrankung, Schmerz, einer neurologischen Störung, einer psychiatrischen Störung, Kopfverletzung, Autoimmunerkrankung, Entzündung, Abstinenz, Stoffwechselstörung, Steatohepatitis und einer beliebigen Kombination davon.

13. Verbindung, Salz, Stereoisomer, Solvat oder Tautomer zur Verwendung nach Anspruch 12, wobei der Tumor ausgewählt ist aus der Gruppe bestehend aus:
Kolorektalkrebs, Brustkrebs, Eierstockkrebs, Bauchspeicheldrüsenkrebs, Leberkrebs und einer beliebigen Kombination davon und/oder wobei die neurodegenerative Erkrankung ausgewählt ist aus der Gruppe bestehend aus: Alzheimer-Krankheit, Parkinson-Krankheit, Huntington-Krankheit, einer demyelinisierenden Krankheit, wie Multipler Sklerose,
amyotropher Lateralsklerose und akuter disseminierter Enzephalomyelitis, und einer beliebigen Kombination davon und/oder wobei der Schmerz akut oder chronisch ist und/oder wobei die neurologische Störung ausgewählt ist aus der Gruppe bestehend aus: Epilepsie, Migräne, Neurotoxizität und Schlaganfall und/oder wobei die psychiatrische Störung Angst oder Depression ist und/oder wobei die Stoffwechselstörung das metabolische Syndrom ist und/oder wobei die Steatohepatitis nicht-alkoholische Steatohepatitis ist.

14. Pharmazeutische Zusammensetzung, umfassend:
- die Verbindung, das Salz, das Stereoisomer, das Solvat oder das Tautomer, wie in einem der Ansprüche 1 bis 8 definiert,
- einen pharmazeutisch akzeptablen Träger und
- gegebenenfalls ein weiteres therapeutisches Mittel.

15. Pharmazeutische Zusammensetzung nach Anspruch 14 zur Verwendung bei der Vorbeugung und/oder Behandlung eines Zustands nach Anspruch 12 oder 13.

16. Verfahren zur Herstellung einer Verbindung der Formel (I), eines Salzes, Stereoisomers, Solvats oder Tautomers, wie in einem der Ansprüche 1 bis 8 definiert, worin R und/oder R₁ für H stehen, umfassend den Schritt der Entschützung einer Verbindung der Formel (VI): 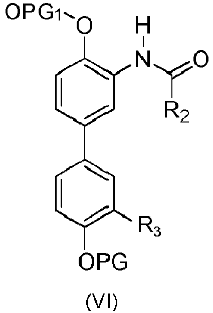 wobei PG und PG₁ die gleichen oder unterschiedliche Schutzgruppen sind.

## Revendications

1. Composé de formule (I) : 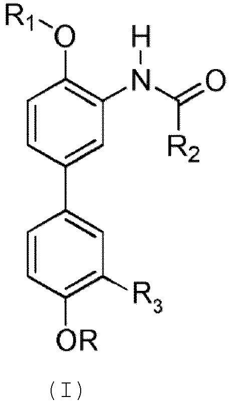 dans lequel :
R et R₁ sont indépendamment sélectionnés dans le groupe constitué par : H, une chaîne alkyle en C1-C6 linéaire, un benzyle et un acétyle ;
R₂ est sélectionné dans le groupe constitué par : une chaîne alkyle en C1-C20 linéaire, une chaîne alkyle en C3-C10 ramifiée, un aryle, un hétéroaryle et une chaîne alkyle en C1-C10 linéaire substituée par un aryle ou un hétéroaryle ;
R₃ est sélectionné dans le groupe constitué par un halogène, un halogénoalkyle et CN ;
les deux cycles présentés sont facultativement et indépendamment l'un de l'autre remplacés par un ou plusieurs substituants indépendamment sélectionnés dans le groupe constitué par : un halogène, une chaîne alkyle en C1-C6 linéaire ou ramifiée, un halogénoalkyle, un halogénoalcoxyle, CN, NO₂, NH₂, un ester carboxylique, COOH et SH ;
ou un sel, un stéréoisomère, un solvate ou un tautomère de celui-ci.

2. Composé, sel, stéréoisomère, solvate ou tautomère selon la revendication 1, dans lequel R est H.

3. Composé, sel, stéréoisomère, solvate ou tautomère selon la revendication 1 ou 2, dans lequel R₃ est sélectionné dans le groupe constitué par : F, Cl, Br, CF₃ et CN.

4. Composé, sel, stéréoisomère, solvate ou tautomère selon l'une quelconque des revendications précédentes, dans lequel R₃ est F.

5. Composé, sel, stéréoisomère, solvate ou tautomère selon l'une quelconque des revendications précédentes, dans lequel R est H et R₃ est F.

6. Composé, sel, stéréoisomère, solvate ou tautomère selon l'une quelconque des revendications précédentes, dans lequel R₁ est H.

7. Composé, sel, stéréoisomère, solvate ou tautomère selon l'une quelconque des revendications précédentes, dans lequel R₂ est sélectionné dans le groupe constitué par : un n-butyle, un n-pentyle, un n-hexyle, un n-heptyle, un n-octyle, un n-nonyle, un n-undécanyle, un n-tridécanyle, un n-pentadécanyle, un isopropyle, un tert-butyle, un phényle, un benzyle, un (CH₂)₂phényle, un (CH₂)₃phényle, un (CH₂)₄phényle et un (CH₂)naphtyle.

8. Composé selon l'une quelconque des revendications précédentes étant sélectionné dans le groupe constitué par :
| | | | |
|---|---|---|---|
| 1 | 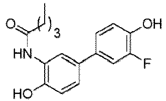 | 9 | 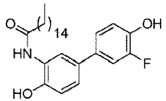 |
| 2 | 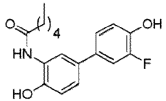 | 10 | 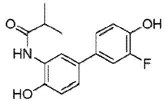 |
| 3 | 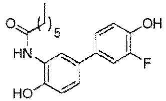 | 11 | 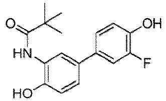 |
| 4 | 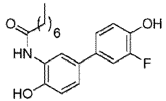 | 12 | 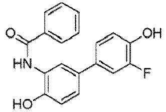 |
| 5 | 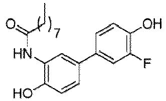 | 13 | 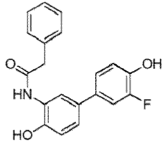 |
| 6 | 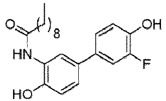 | 14 | 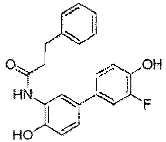 |
| 7 | 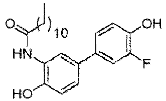 | 15 | 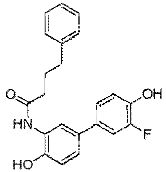 |
| 8 | 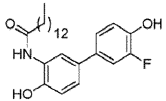 | 16 | 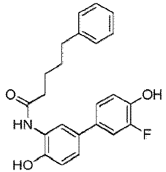 |
| | 17 | 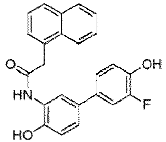 | |
ou un sel, un stéréoisomère, un solvate ou un tautomère de celui-ci.

9. Composé, sel, stéréoisomère, solvate ou tautomère selon l'une quelconque des revendications précédentes pour son utilisation en tant que médicament.

10. Composé, sel, stéréoisomère, solvate ou tautomère selon l'une quelconque des revendications précédentes pour son utilisation en tant qu'inhibiteur de l'enzyme monoacylglycérol lipase (MAGL).

11. Composé, sel, stéréoisomère, solvate ou tautomère pour son utilisation selon la revendication 10, dans lequel cet inhibiteur de l'enzyme MAGL est réversible.

12. Composé, sel, stéréoisomère, solvate ou tautomère selon l'une quelconque des revendications précédentes pour son utilisation dans la prévention et/ou le traitement d'une affection sélectionnée dans le groupe constitué par : une tumeur, une neuroinflammation, une maladie neurodégénérative, une douleur, un trouble neurologique, un trouble psychiatrique, une lésion de la tête, une maladie auto-immune, une inflammation, l'abstinence, un trouble métabolique, une stéatohépatite, et toute combinaison de ceux-ci.

13. Composé, sel, stéréoisomère, solvate ou tautomère pour son utilisation selon la revendication 12, dans lequel ladite tumeur est sélectionnée dans le groupe constitué par : un cancer colorectal, un cancer du sein, un cancer de l'ovaire, un cancer du pancréas, un cancer du foie et toute combinaison de ceux-ci et/ou dans lequel ladite maladie neurodégénérative est sélectionnée dans le groupe constitué par : la maladie d'Alzheimer, la maladie de Parkinson, la maladie d'Huntington, une maladie démyélinisante telle que la sclérose en plaques, la sclérose latérale amyotrophique et l'encéphalomyélite disséminée aigüe, et toute combinaison de celles-ci et/ou dans lequel ladite douleur est aigüe ou chronique et/ou dans lequel ledit trouble neurologique est sélectionné dans le groupe constitué par : l'épilepsie, la migraine, la neurotoxicité et un accident vasculaire cérébral et/ou dans lequel ledit trouble psychiatrique est l'anxiété ou la dépression et/ou dans lequel ledit trouble métabolique est un syndrome métabolique et/ou ladite stéatohépatite est une stéatohépatite non alcoolique.

14. Composition pharmaceutique comprenant :
- le composé, le sel, le stéréoisomère, le solvate ou le tautomère selon l'une quelconque des revendications 1 à 8,
- un vecteur pharmaceutiquement acceptable, et
- facultativement un autre agent thérapeutique.

15. Composition pharmaceutique selon la revendication 14 pour son utilisation dans la prévention et/ou le traitement d'une affection telle que définie dans la revendication 12 ou 13.

16. Procédé de préparation d'un composé de formule (I), d'un sel, d'un stéréoisomère, d'un solvate ou d'un tautomère selon l'une quelconque des revendications 1 à 8 dans lequel R et/ou R₁ sont H comprenant l'étape de déprotection d'un composé de formule (VI) : 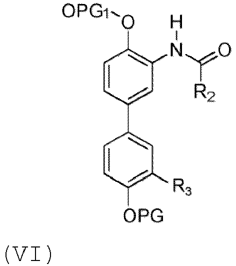 dans lequel PG et PG₁ sont des groupes de déprotection identiques ou différents.
